(19) Europäisches Patentamt
European Patent Office
Office européen des brevets

(11) Veröffentlichungsnummer: **0 602 465 A1**

(12) **EUROPÄISCHE PATENTANMELDUNG**

(21) Anmeldenummer: **93119425.2**

(51) Int. Cl.5: **A01N 43/52**, A61K 31/415

(22) Anmeldetag: **02.12.93**

(30) Priorität: **15.12.92 DE 4242183**

(43) Veröffentlichungstag der Anmeldung:
**22.06.94 Patentblatt 94/25**

(84) Benannte Vertragsstaaten:
**AT BE CH DE DK ES FR GB GR IT LI NL SE**

(71) Anmelder: **BAYER AG**
**D-51368 Leverkusen(DE)**

(72) Erfinder: **Lunkenheimer, Winfried, Dr.**
**Funckstrasse 49**
**D-42115 Wuppertal(DE)**
Erfinder: **Haberkorn, Axel, Prof. Dr.**
**Fuhlrottstrasse 99**
**D-42119 Wuppertal(DE)**

(54) **Verwendung von CN-substituierten Benzimidazolen.**

(57) Die Erfindung betrifft die Verwendung von CN-substituierten Benzimidazolen der allgemeinen Formel (I)

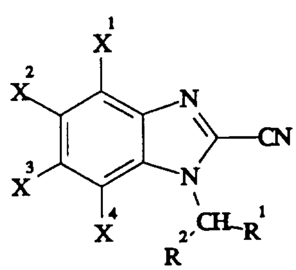

(I)

$X^1$, $X^2$, $X^3$ und $X^4$ unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen,
wobei jedoch mindestens einer der Substituenten$X^1$, $X^2$, $X^3$ und $X^4$ verschieden von Wasserstoff und Halogen ist und wobei

$R^1$ für Wasserstoff, Alkyl oder für gegebenenfalls substituiertes Aryl steht,

$R^2$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Alkylcarbonyl, Alkoxycarbonyl, Alkylcarbonyloxy, Dialkoxyphosphoryl, (Hetero)Aryl, (Hetero)Arylcarbonyl, (Hetero)-Aryloxycarbonyl, (Hetero)Arylcarbonyloxy oder (Hetero)-Arylaminocarbonylaminocarbonyloxy steht,

als Mittel zur Bekämpfung parasitärer Protozoen und insbesondere Coccidien.

EP 0 602 465 A1

Die vorliegende Erfindung betrifft die Verwendung von CN-substituierten Benzimidazolen als Mittel zur Bekämpfung parasitärer Protozoen und insbesondere Coccidien sowie Fisch- und Insektenparasiten.

Substituierte Benzimidazole und ihre Verwendung als Insektizide, Fungizide und Herbizide sind bereits bekannt geworden (EP-OS 87 375, 152 360, US-P 3 472 865, 3 576 818, US-P 3 418 318, EP-OS 260 744, 266 984, 181 826, 239 508).

Polyhalogenierte Benzimidazole und ihre Wirkung als Anthelmintika Coccidiostatika und Pesticide sind bekannt geworden (DE-OS 2 047 369). Doch befriedigt ihre Wirkung nicht in jedem Fall.

Gegenstand der vorliegenden Erfindung ist die Verwendung von CN-substituierten Benzimidazolen der Formel (I)

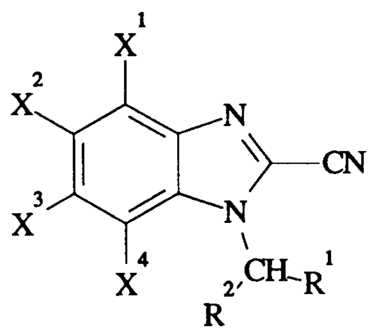

(I)

in welcher

$X^1$, $X^2$, $X^3$ und $X^4$ unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen,

wobei jedoch mindestens einer der Substituenten $X^1$, $X^2$, $X^3$ und $X^4$ verschieden von Wasserstoff und Halogen ist und wobei

$R^1$ für Wasserstoff, Alkyl, Cycloalkyl oder für gegebenenfalls substituiertes Aryl steht,

$R^2$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Aminocarbonyl, Alkylcarbonyl, Alkoxycarbonyl, Alkylcarbonyloxy, Dialkoxyphosphoryl, (Hetero)Aryl, (Hetero)Arylcarbonyl, (Hetero)Aryloxycarbonyl, (Hetero)Arylcarbonyloxy oder (Hetero)-Arylaminocarbonylaminocarbonyloxy steht,

als Mittel zur Bekämpfung parasitärer Protozoen und insbesondere Coccidien.

Die Verbindungen der Formel (I) können gegebenenfalls in Abhängigkeit von der Art und Anzahl der Substituenten als geometrische und/oder optische Isomere bzw. Regioisomere oder deren Isomerengemische in unterschiedlicher Zusammensetzung vorliegen. Sowohl die Verwendung der reinen Isomeren als auch die der Isomerengemische werden erfindungsgemäß beansprucht.

Die erfindungsgemäß verwendbaren substituierten Benzimidazole sind durch die Formel (I) allgemein definiert. Bevorzugt sind Verbindungen der Formel (I), bei welchen

$X^1$, $X^2$, $X^3$ und $X^4$ unabhängig voneinander jeweils für Wasserstoff, Fluor, Chlor, Brom, Iod, Cyano, Nitro, für jeweils geradkettiges oder verzweigtes Alkyl, Alkoxy, Alkylthio, Alkylsulfinyl oder Alkylsulfonyl mit jeweils 1 bis 8 Kohlenstoffatomen, für Cycloalkyl mit 3 bis 8 Kohlenstoffatomen, für jeweils geradkettiges oder verzweigtes Halogenalkyl, Halogenalkoxy, Halogenalkylthio, Halogenalkylsulfinyl, Halogenalkylsulfonyl mit jeweils 1 bis 6 Kohlenstoffatomen und 1 bis 13 gleichen oder verschiedenen Halogenatomen oder für gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkrylen mit 1 bis 5 Kohlenstoffatomen stehen, außerdem für Hydroxycarbonyl, für jeweils geradkettiges oder verzweigtes Alkylcarbonyl oder Alkoxycarbonyl mit jeweils 1 bis 6 Kohlenstoffatomen im Alkylteil, für Cycloalkyloxycarbonyl mit 3 bis 8 Kohlenstoffatomen im Cycloalkylteil oder für jeweils gegebenenfalls einfach oder mehrfach, gleich oder verschieden substituiertes Amino oder Aminocarbonyl stehen, wobei als Aminosubstituenten jeweils infrage kommen:

jeweils geradkettiges oder verzweigtes Alkyl mit 1 bis 6 Kohlenstoffatomen, Halogenalkyl mit 1 bis 6 Kohlenstoffatomen und 1 bis 13 Halogenatomen, Alkoxyalkyl oder Alkylcarbonyl mit jeweils 1 bis 6 Kohlenstoffatomen in den einzelnen Alkylteilen oder im Arylteil jeweils gegebenenfalls einfach oder mehrfach, gleich oder verschieden substituiertes Arylcarbonyl, Arylsulfonyl, Arylaminocarbonyl oder Arylmethylsulfonyl mit jeweils 6 bis 10 Kohlenstoffatomen im Arylteil, wobei als Arylsubstituenten jeweils die bei $R^1$ genannten infrage kommen:

außerdem für jeweils gegebenenfalls im Arylteil einfach oder mehrfach, gleich oder verschieden substituiertes Aryl, Aryloxy, Arylthio, Arylsulfinyl, Arylsulfonyl, Arylsulfonyloxy, Arylcarbonyl, Aryloxycarbonyl, Arylthiomethylsulfonyl oder Arylazo mit jeweils 6 bis 10 Kohlenstoffatomen im Arylteil stehen, wobei als Arylsubstituenten jeweils die bei $R^1$ genannten infrage kommen infrage kommen,

wobei jedoch mindestens einer der Substituenten $X^1$, $X^2$, $X^3$ und $X^4$ verschieden von Wasserstoff und Halogen ist und wobei

$R^1$ für Wasserstoff, für geradkettiges oder verzweigtes Alkyl mit 1 bis 8 Kohlenstoffatomen oder für gegebenenfalls einfach oder mehrfach, gleich oder verschieden substituiertes Phenyl steht, wobei als Substituenten infrage kommen:

Halogen, Cyano, Nitro, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 6 Kohlenstoffatomen in den einzelnen Alkylteilen, gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes, zweifach verknüpftes Dioxyalkylen mit 1 bis 5 Kohlenstoffatomen oder gegebenenfalls einfach oder mehrfach, gleich oder verschieden durch Halogen und/oder geradkettiges oder verzweigtes Alkyl mit 1 bis 6 Kohlenstoffatomen und/oder geradkettiges oder verzweigtes Halogenalkyl mit 1 bis 6 Kohlenstoffatomen und 1 bis 13 gleichen oder verschiedenen Halogenatomen substituiertes Phenyl,

$R^2$ für Hydroxy, Cyano oder für jeweils gegebenenfalls einfach oder mehrfach, gleich oder verschieden substituiertes Alkyl, Alkenyl, Alkinyl, Alkoxy, Alkenyloxy, Alkinyloxy, Alkylthio, Alkylcarbonyl, Alkoxycarbonyl, Alkylcarbonyloxy oder Dialkoxyphosphoryl mit jeweils bis zu 8 Kohlenstoffatomen in den einzelnen Alkyl bzw. Alkenyl oder Alkinylteilen steht, wobei als Substituenten jeweils infrage kommen:

Halogen, geradkettiges oder verzweigtes Alkoxy mit 1 bis 8 Kohlenstoffatomen oder gegebenenfalls einfach oder mehrfach, gleich oder verschieden substituiertes Aryl mit 6 bis 10 Kohlenstoffatomen, wobei als Arylsubstiuenten die bei $R^1$ genannten infrage kommen,

$R^2$ außerdem für jeweils gegebenenfalls einfach oder zweifach, gleich oder verschieden substituiertes Amino oder Aminocarbonyl steht, wobei als Substituenten infrage kommen:

geradkettiges oder verzweigtes Alkyl mit 1 bis 8 Kohlenstoffatomen, geradkettiges oder verzweigtes Alkenyl mit 2 bis 8 Kohlenstoffatomen, Cycloalkyl mit 3 bis 8 Kohlenstoffatomen, Alkoxycarbonyl, Alkylthio-carbonyl, Alkoxythiocarbonyl oder Alkylthio-thiocarbonyl mit jeweils 1 bis 8 Kohlenstoffatomen in den einzelnen geradkettigen oder verzweigten Alkylteilen, zweifach verknüpftes, ringgeschlossenes Alkandiyloxycarbonyl mit 2 bis 6 Kohlenstoffatomen im Alkandiylteil oder jeweils gegebenenfalls einfach oder mehrfach, gleich oder verschieden substituiertes Arylalkyl oder Aryl mit jeweils 6 bis 10 Kohlenstoffatomen im Arylteil und gegebenenfalls 1 bis 6 Kohlenstoffatomen im gerdkettigen oder verzweigten Alkylteil, wobei als Arylsubstituenten jeweils die bei $R^1$ genannten infrage kommen,

$R^2$ außerdem für jeweils gegebenenfalls einfach oder mehrfach, gleich oder verschieden substituiertes Aryl, Arylcarbonyl, Aryloxycarbonyl, Arylcarbonyloxy oder Arylaminocarbonylaminocarbonyloxy mit jeweils 6 bis 10 Kohlenstoffatomen im Arylteil steht, wobei als Arylsubstituenten jeweils die bei $R^1$ genannten infrage kommen,

$R^2$ außerdem für jeweils gegebenenfalls einfach oder mehrfach, gleich oder verschieden substituiertes Heteroaryl, Heteroarylcarbonyl, Heteroaryloxycarbonyl, Heteroarylcarbonyloxy oder Heteroarylaminocarbonylaminocarbonyloxy mit jeweils 2 bis 9 Kohlenstoffatomen und 1 bis 5 gleichen oder verschiednen Heteroatomen - insbesondere Stickstoff, Sauerstoff und/oder Schwefel - im Heteroarylteil steht, wobei als Heteroarylsubstituenten jeweils die bei $R^1$ genannten Arylsubstituenten infrage kommen.

Besonders bevorzugt sind Verbindungen der Formel (I), bei welchen

$X^1$, $X^2$, $X^3$ und $X^4$ unabhängig voneinander jeweils für Wasserstoff, Fluor, Chlor, Brom, Cyano, Nitro, für jeweils geradkettiges oder verzweigtes Alkyl, Alkoxy, Alkylthio, Alkylsulfinyl oder Alkylsulfonyl mit jeweils 1 bis 6 Kohlenstoffatomen, für Cycloalkyl mit 3 bis 7 Kohlenstoffatomen, für jeweils geradkettiges oder verzweigtes Halogenalkyl, Halogenalkoxy, Halogenalkylthio, Halogenalkylsulfinyl, Halogenalkylsulfonyl mit jeweils 1 bis 4 Kohlenstoff-atomen und 1 bis 9 gleichen oder verschiedenen Halogenatomen oder für gegebenenfalls einfach bis sechsfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, zweifach verknüpftes Dioxyalkylen mit 1 bis 4 Kohlenstoffatomen stehen, außerdem für Hydroxycarbonyl, für jeweils geradkettiges oder verzweigtes Alkylcarbonyl oder Alkoxycarbonyl mit jeweils 1 bis 4 Kohlenstoffatomen im Alkylteil, für Cycloalkyloxycarbonyl mit 3 bis 7 Kohlenstoffatomen im Cycloalkylteil oder für jeweils gegebenenfalls einfach oder zweifach, gleich oder verschieden substituiertes Amino oder Aminocarbonyl stehen, wobei als Aminosubstituenten jeweils infrage kommen:
jeweils geradkettiges oder verzweigtes Alkyl mit 1 bis 4 Kohlenstoffatomen, Halogenalkyl mit 1 bis 4 Kohlenstoffatomen und 1 bis 9 Halogenatomen, Alkoxyalkyl oder Alkylcarbonyl mit jeweils 1 bis 4 Kohlenstoffatomen in den einzelnen Alkylteilen oder im Arylteil jeweils gegebenenfalls einfach bis fünffach, gleich oder verschieden substituiertes Arylcarbonyl, Arylsulfonyl, Arylaminocarbonyl oder Arylmethylsulfonyl mit jeweils 6 oder 10 Kohlenstoffatomen im Arylteil, wobei als Arylsubstituenten jeweils die bei $R^1$ genannten infrage kommen:
außerdem für jeweils gegebenenfalls im Arylteil einfach bis fünffach, gleich oder verschieden substituiertes Aryl, Aryloxy, Arylthio, Arylsulfinyl, Arylsulfonyl, Arylsulfonyloxy, Arylcarbonyl, Aryloxycarbonyl, Arylthiomethylsulfonyl oder Arylazo mit jeweils 6 oder 10 Kohlenstoffatomen im Arylteil stehen, wobei als Arylsubstituenten jeweils die bei $R^1$ genannten infrage kommen infrage kommen,
wobei jedoch mindestens einer der Substituenten $X^1$, $X^2$, $X^3$ und $X^4$ verschieden von Wasserstoff und Halogen ist und wobei

$R^1$ für Wasserstoff, für geradkettiges oder verzweigtes Alkyl mit 1 bis 6 Kohlenstoffatomen oder für gegebenenfalls einfach bis dreifach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 4 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 4 Kohlenstoffatomen und 1 bis 9 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 4 Kohlenstoffatomen in den einzelnen Alkylteilen, gegebenenfalls einfach bis sechsfach, gleich oder verschieden
durch Halogen und/oder geradkettiges oder verzweigtes Alkyl mit 1 bis 4 Kohlenstoffatomen und/oder geradkettiges oder verzweigtes Halogenalkyl mit 1 bis 4

Kohlenstoffatomen und 1 bis 9 gleichen oder verschiedenen Halogenatomen substituiertes, zweifach verknüpftes Dioxyalkylen mit 1 bis 4 Kohlenstoffatomen oder gegebenenfalls einfach bis fünffach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes Phenyl,

$R^2$ für Hydroxy, Cyano oder für jeweils gegebenenfalls einfach bis fünffach, gleich oder verschieden durch Halogen substituiertes Alkyl, Alkenyl, Alkinyl, Alkoxy, Alkenyloxy, Alkinyloxy, Alkylthio, Alkylcarbonyl, Alkoxycarbonyl, Alkylcarbonyloxy oder Dialkoxyphosphoryl mit jeweils bis zu 6 Kohlenstoffatomen in den einzelnen Alkyl bzw. Alkenyl oder Alkinylteilen steht, oder für jeweils gegebenenfalls einfach bis dreifach, gleich oder verschieden substituiertes Alkyl, Alkenyl, Alkinyl, Alkoxy, Alkenyloxy, Alkinyloxy, Alkylthio, Alkylcarbonyl, Alkoxycarbonyl, Alkylcarbonyloxy oder Dialkoxyphosphoryl mit jeweils bis zu 6 Kohlenstoffatomen in den einzelnen Alkyl bzw. Alkenyl oder Alkinylteilen steht,wobei als Substituenten jeweils infrage kommen:

geradkettiges oder verzweigtes Alkoxy mit 1 bis 6 Kohlenstoffatomen oder gegebenenfalls einfach bis dreifach, gleich oder verschieden substituiertes Aryl mit 6 oder 10 Kohlenstoffatomen, wobei als Arylsubstiuenten die bei $R^1$ genannten infrage kommen,

$R^2$ außerdem für jeweils gegebenenfalls einfach oder zweifach, gleich oder verschieden substituiertes Amino oder Aminocarbonyl steht, wobei als Substituenten infrage kommen:

geradkettiges oder verzweigtes Alkyl mit 1 bis 6 Kohlenstoffatomen, geradkettiges oder verzweigtes Alkenyl mit 2 bis 6 Kohlenstoffatomen, Cycloalkyl mit 3 bis 7 Kohlenstoffatomen, Alkoxycarbonyl, Alkylthio-carbonyl, Alkoxy-thiocarbonyl oder Alkylthio-thiocarbonyl mit jeweils 1 bis 6 Kohlenstoffatomen in den einzelnen geradkettigen oder verzweigten Alkylteilen, zweifach verfnüpftes, ringgeschlossenes Alkandiyloxycarbonyl mit 2 bis 5 Kohlenstoffatomen im Alkandiylteil oder jeweils gegebenenfalls einfach bis dreifach, gleich oder verschieden substituiertes Arylalkyl oder Aryl mit jeweils 6 oder 10 Kohlenstoffatomen im Arylteil und gegebenenfalls 1 bis 6 Kohlenstoffatomen im geradkettigen oder verzweigten Alkylteil, wobei als Arylsubstituenten jeweils die bei $R^1$ genannten infrage kommen,

$R^2$ außerdem für jeweils gegebenenfalls einfach bis fünffach, gleich oder verschieden substituiertes Aryl, Arylcarbonyl, Aryloxycarbonyl, Arylcarbonyloxy oder Arylaminocarbonylaminocarbonyloxy mit jeweils 6 oder 10 Kohlenstoffatomen im Arylteil steht, wobei als Arylsubstituenten jeweils die bei $R^1$ genannten infrage kommen,

$R^2$ außerdem für jeweils gegebenenfalls einfach bis fünffach, gleich oder verschieden substituiertes Heteroaryl, Heteroarylcarbonyl, Heteroaryloxycarbonyl, Heteroarylcarbonyloxy oder Heteroarylaminocarbonylaminocarbonyloxy mit jeweils 2 bis 9 Kohlenstoffatomen und 1 bis 4 gleichen oder verschiedenen Heteroatomen - insbesondere Stickstoff, Sauerstoff und/oder Schwefel - im Heteroarylteil steht, wobei als Heteroarylsubstituenten jeweils die bei $R^1$ genannten Arylsubstituenten infrage kommen.

Als Arylreste seien genannt Phenyl oder Naphthyl, als Heteroarylreste seien genannt Pyridyl.

Ganz besonders bevorzugt sind Verbindungen der Formel (I), bei welchen

$X^1$, $X^2$, $X^3$ und $X^4$ unabhängig voneinander jeweils für Wasserstoff, Chlor, Brom, Cyano, Nitro, für jeweils geradkettiges oder verzweigtes Alkyl, Alkoxy, Alkylthio, Alkylsulfinyl oder Alkylsulfonyl mit jeweils 1 bis 4 Kohlenstoffatomen, für Cycloalkyl mit 3, 5 oder 6 Kohlenstoffatomen, für jeweils geradkettiges oder verzweigtes Halogenalkyl, Halogenalkoxy, Halogenalkylthio, Halogenalkylsulfinyl, Halogenalkylsulfonyl mit jeweils 1 bis 3 Kohlenstoffatomen und 1 bis 7 gleichen oder verschiedenen Halogenatomen oder für gegebenenfalls einfach bis vierfach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen substituiertes, zweifach verknüpftes Dioxyalkylen mit 1 bis 3 Kohlenstoffatomen stehen, außerdem

für Hydroxycarbonyl, für jeweils geradkettiges oder verzweigtes Alkylcarbonyl oder Alkoxycarbonyl mit jeweils 1 bis 3 Kohlenstoffatomen im Alkylteil, für Cycloalkyloxycarbonyl mit 3,5 oder 6 Kohlenstoffatomen im Cycloalkylteil oder für jeweils gegebenenfalls einfach oder zweifach, gleich oder verschieden substituiertes Amino oder Aminocarbonyl stehen, wobei als Aminosubstituenten jeweils infrage kommen:
jeweils geradkettiges oder verzweigtes Alkyl mit 1 bis 3 Kohlenstoffatomen, Halogenalkyl mit 1 bis 3 Kohlenstoffatomen und 1 bis 7 Halogenatomen, Alkoxyalkyl oder Alkylcarbonyl mit jeweils 1 bis 3 Kohlenstoffatomen in den einzelnen Alkylteilen oder im Phenylteil jeweils gegebenenfalls einfach bis dreifach, gleich oder verschieden substituiertes Phenylcarbonyl, Phenylsulfonyl, Phenylaminocarbonyl oder Phenylmethylsulfonyl, wobei als Phenylsubstituenten jeweils die bei $R^1$ genannten infrage kommen;
außerdem für jeweils gegebenenfalls im Phenylteil einfach bis dreifach, gleich oder verschieden substituiertes Phenyl, Phenyloxy, Phenylthio, Phenylsulfinyl, Phenylsulfonyl, Phenylsulfonyloxy, Phenylcarbonyl, Phenyloxycarbonyl, Phenylthiomethylsulfonyl oder Phenylazo, wobei als Phenylsubstituenten jeweils die bei $R^1$ genannten infrage kommen infrage kommen,
wobei jedoch mindestens einer der Substituenten $X^1$, $X^2$, $X^3$ oder $X^4$ verschieden von Wasserstoff und Halogen ist und wobei

$R^1$ für Wasserstoff, für geradkettiges oder verzweigtes Alkyl mit 1 bis 4 Kohlenstoffatomen oder für gegebenenfalls einfach oder zweifach, gleich oder verschieden substituiertes Phenyl steht, wobei als Substituenten infrage kommen:
Halogen, Cyano, Nitro, jeweils geradkettiges oder verzweigtes Alkyl, Alkoxy, Alkylthio, Alkylsulfinyl oder Alkylsulfonyl mit jeweils 1 bis 3 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 3 Kohlenstoffatomen und 1 bis 7 gleichen oder verschiedenen Halogenatomen, jeweils geradkettiges oder verzweigtes Alkoxyalkyl, Alkoxyalkoxy, Alkanoyl, Alkoxycarbonyl oder Alkoximinoalkyl mit jeweils 1 bis 3 Kohlenstoffatomen in den einzelnen Alkylteilen, gegebenenfalls einfach bis vierfach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen substituiertes, zweifach verknüpftes Dioxyalkylen mit 1 bis 3 Kohlenstoffatomen oder gegebenenfalls einfach bis dreifach, gleich oder verschieden durch Halogen und/oder geradkettiges oder verzweigtes Alkyl mit 1 bis 3 Kohlenstoffatomen und/oder geradkettiges oder verzweigtes Halogenalkyl mit 1 bis 3 Kohlenstoffatomen und 1 bis 7 gleichen oder verschiedenen Halogenatomen substituiertes Phenyl,

$R^2$ für Hydroxy, Cyano oder für jeweils gegebenenfalls einfach bis dreifach, gleich oder verschieden durch Halogen substituiertes Alkyl, Alkenyl, Alkinyl, Alkoxy, Alkenyloxy, Alkinyloxy, Alkylthio, Alkylcarbonyl, Alkoxycarbonyl, Alkylcarbonyloxy oder Dialkoxyphosphoryl mit jeweils bis zu 4 Kohlenstoffatomen in den einzelnen Alkyl bzw. Alkenyl oder Alkinylteilen steht, oder für jeweils gegebenenfalls einfach oder zweifach, gleich oder verschieden substituiertes Alkyl, Alkenyl, Alkinyl, Alkoxy, Alkenyloxy, Alkinyloxy, Alkylthio, Alkylcarbonyl, Alkoxycarbonyl, Alkylcarbonyloxy oder Dialkoxyphosphoryl mit jeweils bis zu 4 Kohlenstoffatomen in den einzelnen Alkyl bzw. Alkenyl oder Alkinylteilen steht,wobei als Substituenten jeweils infrage kommen:
geradkettiges oder verzweigtes Alkoxy mit 1 bis 3 Kohlenstoffatomen oder gegebenenfalls einfach oder zweifach, gleich oder verschieden substituiertes Phenyl, wobei als Phenylsubstiuenten die bei $R^1$ genannten infrage kommen,

$R^2$ außerdem für jeweils gegebenenfalls einfach oder zweifach, gleich oder verschieden substituiertes Amino oder Aminocarbonyl steht, wobei als Substituenten infrage kommen:
geradkettiges oder verzweigtes Alkyl mit 1 bis 4 Kohlenstoffatomen, geradkettiges oder verzweigtes Alkenyl mit 2 bis 4 Kohlenstoffatomen, Cycloalkyl mit 3 bis 6 Kohlenstoffatomen, Alkoxycarbonyl, Alkylthio-carbonyl, Alkoxy-thiocarbonyl oder Alkylthio-thiocarbonyl mit jeweils 1 bis 4 Kohlenstoffatomen in den einzelnen geradkettigen oder verzweigten Alkylteilen, zweifach verknüpftes, ringgeschlossenes Alkandiyloxycarbonyl mit 2 bis 4 Kohlenstoffatomen irn Alkandiylteil oder jeweils gegebenenfalls einfach oder zweifach, gleich oder verschieden substituiertes Phenylalkyl oder Phenyl mit gegebenenfalls 1 bis 3 Kohlenstoffatomen im geradkettigen oder verzweigten Alkylteil, wobei als Phenylsubstituenten jeweils die bei $R^1$ genannten infrage kommen,

$R^2$ außerdem für jeweils gegebenenfalls einfach bis dreifach, gleich oder verschieden substituiertes Phenyl, Phenylcarbonyl, Phenyloxycarbonyl, Phenylcarbonyloxy oder Phenylaminocarbonylaminocarbonyloxy steht, wobei als Phenylsubstituenten jeweils die bei $R^1$ genannten infrage kommen,

$R^2$ außerdem für jeweils gegebenenfalls einfach bis dreifach, gleich oder verschieden substituiertes Heteroaryl, Heteroarylcarbonyl, Heteroaryloxycarbonyl, Heteroarylcarbonyloxy oder Heteroarylaminocarbonylaminocarbonyloxy mit jeweils 2 bis 9 Kohlenstoffatomen und 1 bis 3 gleichen oder verschiedenen Heteroatomen - insbesondere Stickstoff, Sauerstoff und/oder Schwefel - im Heteroarylteil steht, wobei als Heteroarylsubstituenten jeweils die bei $R^1$ genannten Phenylsubstituenten infrage kommen,

Als Heteroaryl sei genannt Pyridyl.

Im einzelnen seien außer den bei den Herstellungsbeispielen genannten Verbindungen die folgenden substituierten Benzimidazole der allgemeinen Formel (I) genannt:

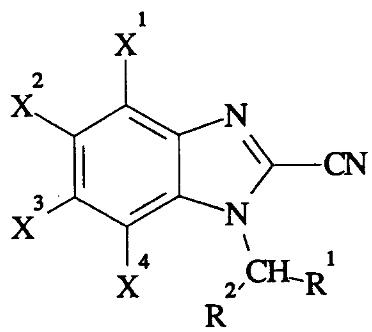

(I)

| $X^1$ | $X^2$ | $X^3$ | $X^4$ | A |
|---|---|---|---|---|
| Br | H | $CF_3$-S- | H | 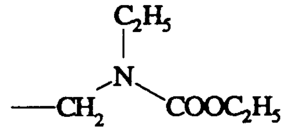 |
| Br | H | $CF_3$-S- | H | $-CH_2-O-C_2H_5$ |
| Br | H | ClFCH-$CF_2$-S- | H | 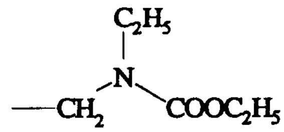 |
| Br | H | ClFCH-$CF_2$-S- | H | $-CH_2-O-C_2H_5$ |
| Br | H | $F_3C$-CHF-$CF_2$-S- | H | 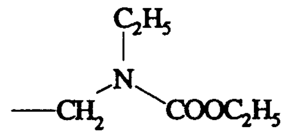 |
| Br | H | $F_3C$-CHF-$CF_2$-S- | H | $-CH_2-O-C_2H_5$ |
| Br | H | $F_3C$- | H | $-CH_2-O-C_2H_5$ |
| Br | H | $F_3C$-O- | H | 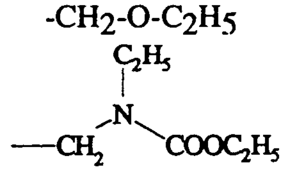 |
| Br | H | $F_3C$-O- | H | $-CH_2-O-C_2H_5$ |
| Br | H | $NO_2$ | H | 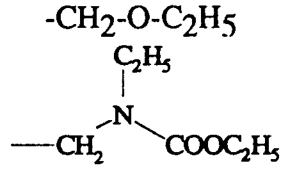 |
| Br | H | $NO_2$ | H | $-CH_2-O-C_2H_5$ |
| Cl | H | $CF_3$-S- | H | 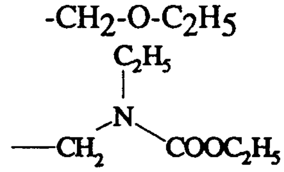 |
| Cl | H | $CF_3$-S- | H | $-CH_2-O-C_2H_5$ |
| Cl | H | ClFCH-$CF_2$-S- | H | 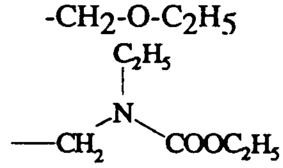 |
| Cl | H | ClFCH-$CF_2$-S- | H | $-CH_2-O-C_2H_5$ |
| Cl | H | $F_3C$-CHF-$CF_2$-S- | H | 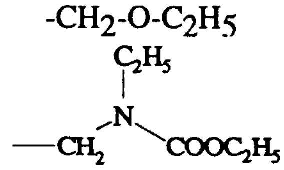 |

| $X^1$ | $X^2$ | $X^3$ | $X^4$ | A |
|---|---|---|---|---|
| Cl | H | $F_3C-CHF-CF_2-S-$ | H | $-CH_2-O-C_2H_5$ |
| Cl | H | $F_3C-$ | H | 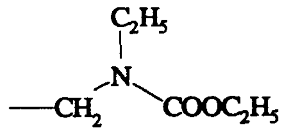 |
| Cl | H | $F_3C-$ | H | $-CH_2-O-C_2H_5$ |
| Cl | H | $F_3C-O-$ | H | 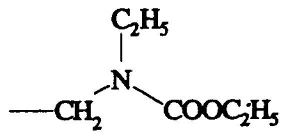 |
| Cl | H | $F_3C-O-$ | H | $-CH_2-O-C_2H_5$ |
| Cl | H | $NO_2$ | H | 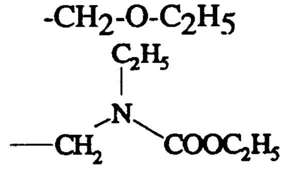 |
| Cl | H | $NO_2$ | H | $-CH_2-O-C_2H_5$ |
| $CF_3$ | H | $CF_3-S-$ | H | 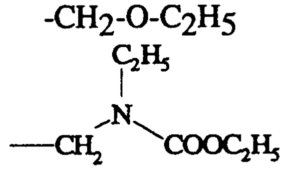 |
| $CF_3$ | H | $CF_3-S-$ | H | $-CH_2-O-C_2H_5$ |
| $CF_3$ | H | $ClFCH-CF_2-S-$ | H | 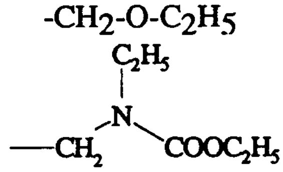 |
| $CF_3$ | H | $ClFCH-CF_2-S-$ | H | $-CH_2-O-C_2H_5$ |
| $CF_3$ | H | $F_3C-CHF-CF_2-S-$ | H | 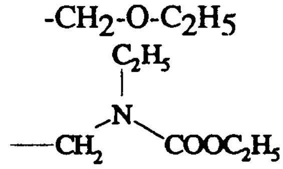 |
| $CF_3$ | H | $F_3C-CHF-CF_2-S-$ | H | $-CH_2-O-C_2H_5$ |
| $CF_3$ | H | $F_3C-$ | H | 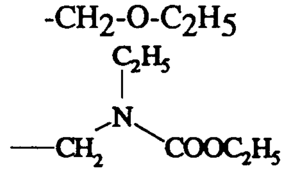 |
| $CF_3$ | H | $F_3C-$ | H | $-CH_2-O-C_2H_5$ |

| $X^1$ | $X^2$ | $X^3$ | $X^4$ | A |
|---|---|---|---|---|
| $CF_3$ | H | $F_3C-O-$ | H | 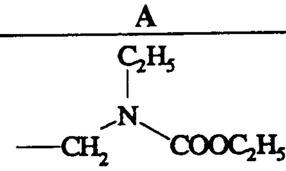 |
| $CF_3$ | H | $F_3C-O-$ | H | $-CH_2-O-C_2H_5$ |
| $CF_3$ | H | $NO_2$ | H | 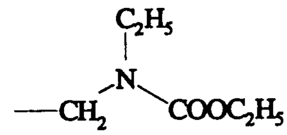 |
| $CF_3$ | H | $NO_2$ | H | $-CH_2-O-C_2H_5$ |
| $COOCH_3$ | H | $CF_3-S-$ | H | 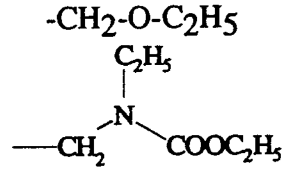 |
| $COOCH_3$ | H | $CF_3-S-$ | H | $-CH_2-O-C_2H_5$ |
| $COOCH_3$ | H | $ClFCH-CF_2-S-$ | H | 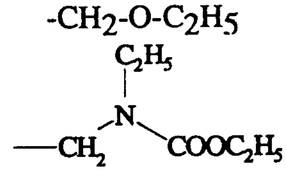 |
| $COOCH_3$ | H | $ClFCH-CF_2-S-$ | H | $-CH_2-O-C_2H_5$ |
| $COOCH_3$ | H | $F_3C-CHF-CF_2-S-$ | H | 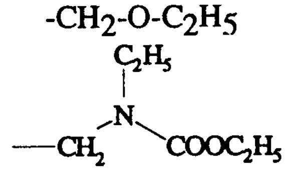 |
| $COOCH_3$ | H | $F_3C-CHF-CF_2-S-$ | H | $-CH_2-O-C_2H_5$ |
| $COOCH_3$ | H | $F_3C-$ | H | 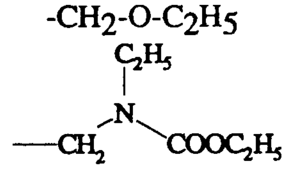 |
| $COOCH_3$ | H | $F_3C-$ | H | $-CH_2-O-C_2H_5$ |
| $COOCH_3$ | H | $F_3C-O-$ | H | 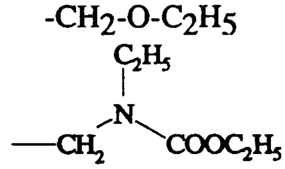 |
| $COOCH_3$ | H | $F_3C-O-$ | H | $-CH_2-O-C_2H_5$ |

| $X^1$ | $X^2$ | $X^3$ | $X^4$ | A |
|---|---|---|---|---|
| $COOCH_3$ | H | $NO_2$ | H | 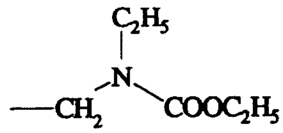 |
| $COOCH_3$ | H | $NO_2$ | H | $-CH_2-O-C_2H_5$ |
| $COO-n-C_3H_7$ | H | $CF_3-S-$ | H | 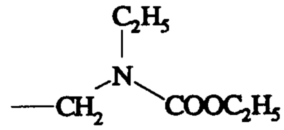 |
| $COO-n-C_3H_7$ | H | $CF_3-S-$ | H | $-CH_2-O-C_2H_5$ |
| $COO-n-C_3H_7$ | H | $ClFCH-CF_2-S-$ | H | 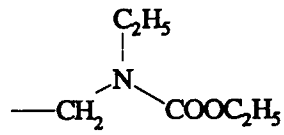 |
| $COO-n-C_3H_7$ | H | $ClFCH-CF_2-S-$ | H | $-CH_2-O-C_2H_5$ |
| $COO-n-C_3H_7$ | H | $F_3C-CHF-CF_2-S-$ | H | 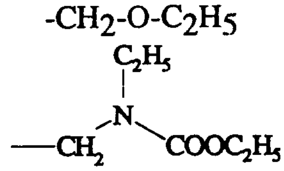 |
| $COO-n-C_3H_7$ | H | $F_3C-CHF-CF_2-S-$ | H | $-CH_2-O-C_2H_5$ |
| $COO-n-C_3H_7$ | H | $F_3C-$ | H | 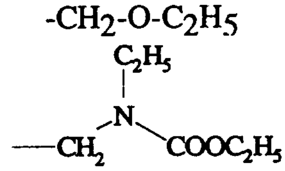 |
| $COO-n-C_3H_7$ | H | $F_3C-$ | H | $-CH_2-O-C_2H_5$ |
| $COO-n-C_3H_7$ | H | $F_3C-O-$ | H | 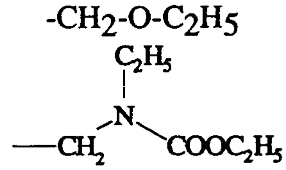 |
| $COO-n-C_3H_7$ | H | $F_3C-O-$ | H | $-CH_2-O-C_2H_5$ |
| $COO-n-C_3H_7$ | H | $NO_2$ | H | 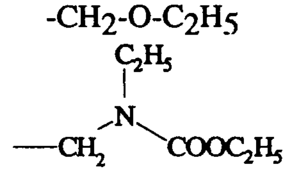 |
| $COO-n-C_3H_7$ | H | $NO_2$ | H | $-CH_2-O-C_2H_5$ |

Die substituierten Benzimidazole der Formel (I) sind teilweise bekannt (vergl. z.B. DE 20 47 369; DE 20 14 293; EP 448 206; J. Chem. Soc. C, 1967, 2536-2540).

Noch nicht bekannt, aber Gegenstand einer älteren Anmeldung der Anmelderin sind substituierte Benzimidazole der Formel (Ia),

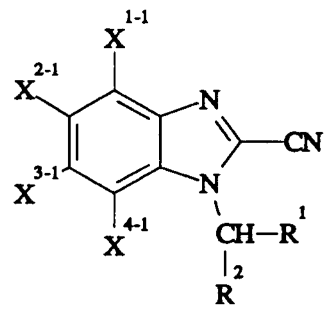

(Ia)

in welcher

$X^{1-1}$, $X^{2-1}$, $X^{3-1}$ und $X^{4-1}$ unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen, wobei jedoch mindestens einer der Substituenten $X^{1-1}$, $X^{2-1}$, $X^{3-1}$ und $X^{4-1}$ für Nitro, Halogenalkyl, Halogenalkoxy, Halogenalkylthio, Halogenalkylsulfinyl, Halogenalkylsulfonyl, Alkylsulfonyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Arylthio, Arylsulfinyl, Arylsulfonyl, Arylsulfonyloxy, Arylcarbonyl, Aryloxycarbonyl, Arylazo oder Arylthiomethylsulfonyl steht und wobei

$R^1$ für Wasserstoff, Alkyl oder für gegebenenfalls substituiertes Aryl steht,

$R^2$ für Hydroxy, Cyano oder für jeweils gegebenenfalls substituiertes Alkyl, Alkenyl, Alkinyl, Alkoxy, Alkenyloxy, Alkinyloxy, Alkylthio, Amino, Alkylcarbonyl, Alkoxycarbonyl, Alkylcarbonyloxy, Dialkoxyphosphoryl, (Hetero)Aryl, (Hetero)Arylcarbonyl, (Hetero)Aryloxycarbonyl, (Hetero)Arylcarbonyloxy oder (Hetero)Arylaminocarbonylaminocarbonyloxy steht.

Man erhält die bekannten und die noch nicht bekannten substituierten Benzimidazole der Formeln (I) bzw. (Ia), wenn man

a) 1H-Benzimidazole der Formel (II),

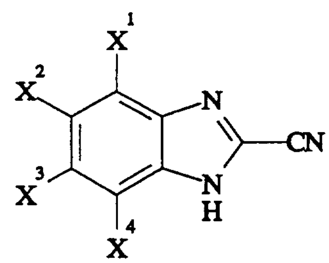

(II)

in welcher

$X^1$, $X^2$, $X^3$ und $X^4$ die oben angegebene Bedeutung haben,

mit Alkylierungsmitteln der Formel (III),

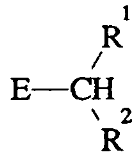

(III)

in welcher

E für eine geeignete Abgangsgruppe steht und

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt.

Verwendet man beispielsweise 5-Nitro-2-cyano-benzimidazol und Chlormethylethylether als Ausgangsverbindungen, so läßt sich der Reaktionsablauf des Herstellungsverfahrens durch das folgende Formelschema darstellen:

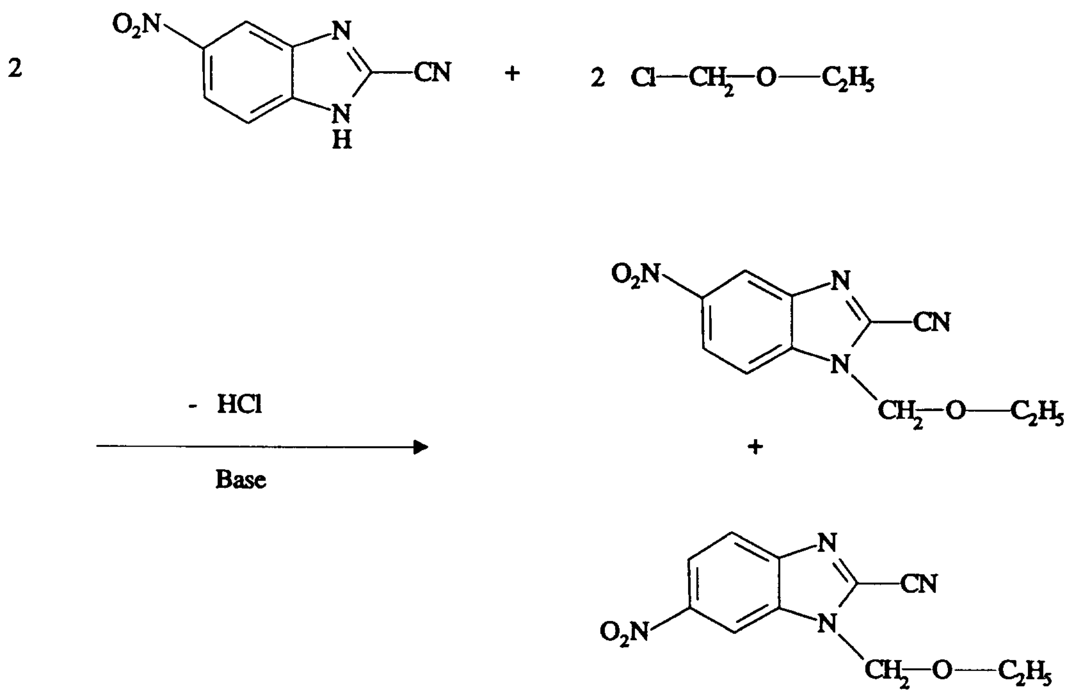


Die zur Durchführung des Herstellungsverfahrens als Ausgangsstoffe benötigten 1H-Benzimidazole sind durch die Formel (II) allgemein definiert. In dieser Formel (II) stehen $X^1$, $X^2$, $X^3$ und $X^4$ vorzugsweise für diejenigen Reste, die bereits im Zusammenhang mit der Beschreibung der erfindungsgemäß verwendbaren Verbindungen der Formel (I) als bevorzugt für diesen Substituenten genannt wurden.

Die 1H-Benzimidazole der Formel (II) sind bekannt oder erhältlich in Analogie zu bekannten Verfahren (vergl. z.B. J. Amer. Chem. Soc. 75, 2192 [1953]; US 3.576.818)

Die zur Durchführung des Herstellungsverfahrens weiterhin als Ausgangsprodukte erforderlichen Alkylierungsmittel sind durch die Formel (III) allgemein definiert. In dieser Formel (III) stehen $R^1$ und $R^2$ vorzugsweise für diejenigen Reste, die bereits im Zusammenhang mit der Beschreibung der erfindungsgemäß verwendbaren Stoffe der Formel (I) als bevorzugt für diesen Substituenten genannt wurden.

E steht für einen bei Alkylierungsmitteln üblichen Abgangsrest, vorzugsweise für Halogen, insbesondere für Chlor, Brom oder Iod oder für jeweils gegebenenfalls substituiertes Alkylsulfonyloxy, Alkoxysulfonyloxy oder Arylsulfonyloxy, wie insbesondere Methansulfonyloxy, Trifluormethansulfonyloxy, Methoxysulfonyloxy, Ethoxysulfonyloxy oder p-Toluolsulfonyloxy.

E steht außerdem auch für eine Alkohol-, Alkanoyloxy- oder Alkoxygruppe, wie beispielsweise eine Hydroxy-, Acetoxy- oder Methoxygruppe, insbesondere wenn mit Hilfe des erfindungsgemäßen Verfahrens

Verbindungen der Formel (I), bei welchen $R^1$ verschieden von Wasserstoff ist, hergestellt werden sollen.

Die Verbindungen der Formel (III) sind allgemein bekannt oder erhältlich in Analogie zu bekannten Verfahren (vergl. z.B. DE 20 40 175; DE 21 19 518; Synthesis 1973, 703).

Als Verdünnungsmittel zur Durchführung des Herstellungverfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform oder Tetrachlorkohlenstoff; Ether, wie Diethylether, Düsopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester oder Basen wie Pyridin oder organische Säuren, wie Ameisensäure oder Essigsäure.

Das Herstellungverfahren wird vorzugsweise in Gegenwart eines geeigneten Reaktionshilfsmittels durchgeführt. Als solche kommen alle üblichen anorganischen oder organischen Basen infrage. Hierzu gehören beispielsweise Erdalkali- oder Alkalimetallhydride, -hydroxide, -amide, -alkoholate, -acetate, -carbonate oder -hydrogencarbonate, wie beispielsweise Natriumhydrid, Natriumamid, Lithium-diethylamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat, Lithium-organische Verbindungen, wie n-Butyllithium sowie tertiäre Amine, wie Trimethylamin, Triethylamin, Tributylamin, Di-isopropyl-ethylamin, Tetramethylguanidin, N,N-Dimethylanilin, Pyridin, Piperidin, N-Methylpiperidin, N,N-Dimethylaminopyridin, Diazabicyclooctan (DABCO), Diazabicyclononen (DBN) oder Diazabicycloundecen (DBU).

Alternativ kommen als Reaktionshilfsmittel auch organische oder anorganische Säuren, wie beispielsweise Schwefelsäure, Salzsäure, p-Toluolsulfonsäure, Perfluorbutansulfonsäure oder stark saure Ionenaustauscher infrage, besonders wenn in den verwendeten Alkylierungsmitteln der Formel (III) E für einen Hydroxy-, Acyloxy- oder Alkoxyrest steht.

Das Herstellungverfahren kann gegebenenfalls auch in einem Zweiphasensystem, wie beispielsweise Wasser/Toluol oder Wasser/Dichlormethan, gegebenenfalls in Gegenwart eines geeigneten Phasentransferkatalysators, durchgeführt werden. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-$C_{13}/C_{15}$-alkylammoniumchlorid, Trimethyl-$C_{13}/C_{15}$-alkylammoniumbromid, Dibenzyl-dimethyl-ammoniummethylsulfat, Dimethyl-$C_{12}/C_{14}$-alkyl-benzylammoniumchlorid, Dimethyl-$C_{12}/C_{14}$-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der Durchführung des Herstellungverfahrens in einem größeren Bereich variiert werden. Im allgemeinen arbeitet man bei Temperaturen zwischen -70°C und +200°C, vorzugsweise bei Temperaturen zwischen 0°C und 130°C.

Das Herstellungverfahren wird üblicherweise unter Normaldruck durchgeführt. Es ist jedoch auch möglich unter erhöhtem oder vermindertem Druck zu arbeiten.

Zur Durchführung des Herstellungverfahrens setzt man pro Mol an 1H-Benzimidazol der Formel (II) im allgemeinen 1,0 bis 5,0 Mol, vorzugsweise 1,0 bis 2,5 Mol an Alkylierungsmittel der Formel (III) und gegebenenfalls 0,01 bis 5,0 Mol, vorzugsweise 1,0 bis 3,0 Mol an Reaktionshilfsmittel ein.

In einer besonderen Durchführungsform ist es auch möglich, die 1H-Benzimidazole der Formel (II) zunächst in einem vorgelagerten Reaktionsschritt mit Hilfe üblicher Silylierungsverfahren beispielsweise mit Hexamethyldisilazan oder Trimethylsilylchlorid, gegebenenfalls in Gegenwart eines geeigneten Katalysators, wie beispielsweise Schwefelsäure, Trifluoressigsäure, Ammoniumsulfat, Imidazol oder Saccharin bei Temperaturen zwischen -20°C und +150°C zu silylieren und die so erhältlichen 1-Trimethylsilylbenzimidazole in einer anschließenden zweiten Stufe mit Alkylierungsmitteln der Formel (II) gemäß dem Herstellungverfahren umzusetzen. In diesem Fall ist es von Vorteil als Katalysator zur Alkylierungsreaktion Zinntetrachlorid zuzusetzen (vergl. z.B. Chem. Heterocycl. Comp. USSR 24, 514 [1988]).

Die Reaktionsdurchführung, Aufarbeitung und Isolierung der Reaktionsprodukte erfolgt nach bekannten Verfahren (vergl. hierzu auch die Herstellungsbeispiele).

Die Reinigung der Endprodukte der Formel (I) erfolgt mit Hilfe üblicher Verfahren, beispielsweise durch Säulenchromatographie oder durch Umkristallisieren.

Die Charakterisierung erfolgt mit Hilfe des Schmelzpunktes oder bei nicht kristallisierenden Verbindungen - insbesondere bei Regioisomerengemischen - mit Hilfe der Protonen-Kernresonanzspektroskopie ($^1$H-NMR).

Die Wirkstoffe eignen sich bei günstiger Warmblütertoxizität zur Bekämpfung von parasitischen Protozoen, die in der Tierhaltung und Tierzucht bei Nutz-, Zucht-, Zoo-, Labor-, Versuchs- und Hobbytieren vorkommen. Sie sind dabei gegen alle oder einzelne Entwicklungsstadien der Schädlinge sowie gegen resistente und normal sensible Stämme wirksam. Durch die Bekämpfung der parasitischen Protozoen sollen Krankheit, Todesfälle und Leistungsminderungen (z.B. bei der Produktion von Fleisch, Milch, Wolle, Häuten, Eiern, Honig usw.) vermindert werden, so daß durch den Einsatz der Wirkstoffe eine wirtschaftlichere und einfachere Tierhaltung möglich ist.

Zu den parasitischen Protozoen zählen:

Mastigophora (Flagellata) wie z.B. Trypanosomatidae z.B. Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie z.B. Trichomonadidae z.B. Giardia lamblia, G. canis.

Sarcomastigophora (Rhizopoda) wie Entamoebidae z.B. Entamoeba histolytica, Hartmanellidae z.B. Acanthamoeba sp., Hartmanella sp.

Apicomplexa (Sporozoa) wie Eimeridae z.B. Eimeria acervulina, E. adenoides, E. alabahmensis, E. anatis, E. anseris, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva,E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec. wie Toxoplasmadidae z.B. Toxoplasma gondii, wie Sarcocystidae z.B. Sarcocystis bovicanis, S. bovihominis, S. ovicanis, S. ovifelis, S. spec., S. suihominis wie Leucozoidae z.B. Leucozytozoon simondi, wie Plasmodüdae z.B. Plasmodium berghei, P. falciparum, P. malariae, P. ovale, P. vivax, P. spec., wie Piroplasmea z.B. Babesia argentina, B. bovis, B. canis, B. spec., Theileria parva, Theileria spec., wie Adeleina z.B. Hepatozoon canis, H. spec.

Ferner Myxospora und Microspora z.B. Glugea spec. Nosema spec.

Ferner Pneumocystis carinii, sowie Ciliophora (Ciliata) wie z.B. Balantidium coli, Ichthiophthirius spec., Trichodina spec., Epistylis spec.

Die erfindungsgemäßen Verbindungen sind auch wirksam gegen Protozoen, die als Parasiten bei Insekten auftreten. Als solche seien genannt Parasiten des Stammes Microsporida, insbesondere der Gattung Nosema. Besonders genannt sei Nosema apis bei der Honigbiene.

Zu den Nutz- und Zuchttieren gehören Säugetiere wie z.B. Rinder, Pferde, Schafe, Schweine, Ziegen, Kamele, Wasserbüffel, Esel, Kaninchen, Damwild, Rentiere, Pelztiere wie z.B. Nerze, Chinchilla, Waschbär, Vögel wie z.B. Hühner, Gänse, Puten, Enten, Tauben, Vogelarten für Heim- und Zoohaltung. Ferner gehören dazu Nutzund Zierfische.

Zu Labor- und Versuchstieren gehören Mäuse, Ratten, Meerschweinchen, Goldhamster, Hunde und Katzen.

Zu den Hobbytieren gehören Hunde und Katzen.

Zu den Fischen gehören Nutz-, Zucht-, Aquarien- und Zierfische aller Altersstufen, die in Süß- und Salzwasser leben. Zu den Nutz- und Zuchtfischen zählen z.B. Karpfen, Aal, Forelle, Weißfisch, Lachs, Brachse, Rotauge, Rotfeder, Döbel, Seezunge, Scholle, Heilbutt, Japanese yellowtail (Seriola quinqueradiata), Japanaal (Anguilla japonica), Red seabraam (Pagurus major), Seabass (Dicentrarchus labrax), Grey mullet (Mugilus cephalus), Pompano, Gilthread seabream (Sparus auratus), Tilapia spp., Chichliden-Arten wie z.B. Plagioscion, Channel catfish. Besonders geeignet sind die erfindungsgemäßen Mittel zur Behandlung von Fischbrut, z.B. Karpfen von 2 bis 4cm Körperlänge. Sehr gut geeignet sind die Mittel auch in der Aalmast.

Die Anwendung kann sowohl prophylaktisch als auch therapeutisch erfolgen.

Die Anwendung der Wirkstoffe erfolgt direkt oder in. Form von geeigneten Zubereitungen enteral, parenteral, dermal, nasal.

Die enterale Anwendung der Wirkstoffe geschieht z.B. oral in Form von Pulver, Zäpfchen, Tabletten, Kapseln, Pasten, Tränken, Granulaten, Drenchen, Boli, medkiertem Futter oder Trinkwasser. Die dermale Anwendung geschieht z.B. in Form des Tauchens (Dippen), Sprühens (Sprayen), Badens, Waschens, Aufgießens (pour-on and spot-on) und desEinpuderns. Die parenterale Anwendung geschieht z.B. in Form der Injektion (intramusculär, subcutan, intravenös, intraperitoneal) oder durch Implantate.

Geeignete Zubereitungen sind:

Lösungen wie Injektionslösungen, orale Lösungen, Konzentrate zur oralen Verabreichung nach Verdünnung, Lösungen zum Gebrauch auf der Haut oder in Körperhöhlen, Aufgußformulierungen, Gele;

Emulsionen und Suspension zur oralen oder dermalen Anwendung sowie zur Injektion; Halbfeste Zubereitungen;
Formulierungen, bei denen der Wirkstoff in einer Salbengrundlage oder in einer Öl in Wasser oder Wasser in Öl Emulsionsgrundlage verarbeitet ist:
Feste Zubereitungen wie Pulver, Premixe oder Konzentrate, Granulate, Pellets, Tabletten, Boli, Kapseln; Aerosole und Inhalate, wirkstoffhaltige Formkörper.

Injektionslösungen werden intravenös, intramuskulär und subcutan verabreicht.

Injektionslösungen werden hergestellt, indem der Wirkstoff in einem geeigneten Lösungsmittel gelöst wird und eventuell Zusätze wie Lösungsvermittler, Säuren, Basen, Puffersalze, Antioxidantien, Konservierungsmittel zugefügt werden. Die Lösungen werden steril filtriert und abgefüllt.

Als Lösungsmittel seien genannt: Physiologisch verträgliche Lösungsmittel wie Wasser, Alkohole wie Ethanol, Butanol, Benzylalkohol, Glycerin, Kohlenwasserstoffe, Propylenglykol, Polyethylenglykole, N-Methylpyrrolidon, sowie Gemische derselben.

Die Wirkstoffe lassen sich gegebenenfalls auch in physiologisch verträglichen pflanzlichen oder synthetischen Ölen, die zur Injektion geeignet sind, lösen.

Als Lösungsvermittler seien genannt: Lösungsmittel, die die Lösung des Wirkstoffs im Hauptlösungsmittel fördern oder sein Ausfallen verhindern. Beispiele sind Polyvinylpyrrolidon, polyoxyethyliertes Rhizinusöl, polyoxyethylierte Sorbitanester.

Konservierungsmittel sind: Benzylalkohol, Trichlorbutanol, p-Hydroxybenzoesäureester, n-Butanol.

Orale Lösungen werden direkt angewendet. Konzentrate werden nach vorheriger Verdünnung auf die Anwendungskonzentration oral angewendet. Orale Lösungen und Konzentrate werden, wie oben bei den Injektionslösungen beschrieben, hergestellt, wobei auf steriles Arbeiten verzichtet werden kann.

Lösungen zum Gebrauch auf der Haut werden aufgeträufelt, aufgestrichen, eingerieben, aufgespritzt, aufgesprüht oder durch Tauchen (Dippen), Baden oder Waschen aufgebracht. Diese Lösungen werden, wie oben bei den Injektionslösungen beschrieben, hergestellt.

Es kann vorteilhaft sein, bei der Herstellung Verdickungsmittel zuzufügen; Verdickungsmittel sind: Anorganische Verdickungsmittel wie Bentonite, kolloidale Kieselsäure, Aluminiummonostearat, organische Verdickungsmittel wie Cellulosederivate, Polyvinylalkohole und deren Copolymere, Acrylate und Metacrylate.

Gele werden auf die aufgetragen oder aufgestrichen oder in Körperhöhlen eingebracht. Gele werden hergestellt, indem Lösungen, die wie bei den Injektionslösungen beschrieben hergestellt worden sind, mit soviel Verdickungsmittel versetzt werden, daß eine klare Masse mit salbenartiger Konsistenz entsteht. Als Verdickungsmittel werden die weiter oben angegebenen Verdickungsmittel eingesetzt.

Aufgießformulierungen werden auf begrenzte Bereiche der Haut aufgegossen oder aufgespritzt, wobei der Wirkstoff entweder die Haut durchdringt und systemisch wirkt oder sich auf der Körperoberfläche verteilt.

Aufgießformulierungen werden hergestellt, indem der Wirkstoff in geeigneten hautverträglichen Lösungsmitteln oder Lösungsmittelgemischen gelöst, suspendiert oder emulgiert wird. Gegebenenfalls werden weitere Hilfsstoffe wie Farbstoffe, resorptionsfördernde Stoffe, Antioxidantien, Lichtschutzmittel, Haftmittel zugefügt.

Als Lösungsmittel seien genannt: Wasser, Alkanole, Glycole, Polyethylenglycole, Polypropylenglycole, Glycerin, aromatische Alkohole wie Benzylalkohol, Phenylethanol, Phenoxyethanol, Ester wie Essigester, Butylacetat, Benzylbenzoat, Ether wie Alkylenglykolalkylether wie Dipropylenglykolmonomethylether, Diethylenglykolmono-butylether, Ketone wie Aceton, Methylethylketon, aromatische und/oder aliphatische Kohlenwasserstoffe, pflanzliche oder synthetische Öle, DMF, Dimethylacetamid, N-Methylpyrrolidon, 2-Dimethyl-4-oxy-methylen-1,3-dioxolan.

Farbstoffe sind alle zur Anwendung am Tier zugelassenen Farbstoffe, die gelöst oder suspendiert sein können.

Resorptionsfördernde Stoffe sind z.B. DMSO, spreitende Öle wie Isopropylmyristat, Dipropylenglykolpelargonat, Silikonöle, Fettsäureester, Triglyceride, Fettalkohole.

Antioxidantien sind Sulfite oder Metabisulfite wie Kaliummetabisulfit, Ascorbinsäure, Butylhydroxytoluol, Butylhydroxyanisol, Tocopherol.

Lichtschutzmittel sind z.B. Stoffe aus der Klasse der Benzophenone oder Novantisolsäure.

Haftmittel sind z.B. Cellulosederivate, Stärkederivate, Polyacrylate, natürliche Polymere wie Alginate, Gelatine.

Emulsionen können oral, dermal oder als Injektionen angewendet werden.

Emulsionen sind entweder vom Typ Wasser in Öl oder von Typ Öl in Wasser.

Sie werden hergestellt, indem man den Wirkstoff entweder in der hydrophoben oder in der hydrophilen Phase löst und diese unter Zuhilfenahme geeigneter Emulgatoren und gegebenenfalls weiterer Hilfsstoffe wie Farbstoffe, resorptionsfördernde Stoffe, Konservierungsstoffe, Antioxidantien, Lichtschutzmittel, viskositätserhöhende Stoffe, mit dem Lösungsmittel der anderen Phase homogenisiert.

Als hydrophobe Phase (Öle) seien genannt: Paraffinöle, Silikonöle, natürliche Pflanzenöle wie Sesamöl, Mandelöl, Rizinusöl, synthetische Triglyceride wie Capryl/Caprinsäure-biglycerid, Triglyceridgemisch mit Pflanzenfettsäuren der Kettenlänge $C_{8-12}$ oder anderen speziell ausgewählten natürlichenFettsäuren, Partialglyceridgemische gesättigter oder ungesättigter, eventuell auch hydroxylgruppenhaltiger Fettsäuren, Mono- und Diglyceride der $C_8/C_{10}$-Fettsäuren.

Fettsäureester wie Ethylstearat, Di-n-butyryl-adipat, Laurinsäurehexylester, Dipropylen-glykolpelargonat, Ester einer verzweigten Fettsäure mittlerer Kettenlänge mit gesättigten Fettalkoholen der Kettenlänge $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester,Ölsäuredecylester, Ethyloleat, Milchsäureethylester, wachsartige Fettsäureester wie Dibutylphthalat, Adipinsäurediisopropylester, letzterem verwandte Estergemische u.a. Fettalkohole wie Isotridecylalkohol, 2-Octyldodecanol, Cetylstearyl-alkohol, Oleylalkohol.

Fettsäuren wie z.B. Ölsäure und ihre Gemische.

Als hydrophile Phase seien genannt:
Wasser, Alkohole wie z.B. Propylenglycol, Glycerin, Sorbitol und ihre Gemische.

Als Emulgatoren seien genannt:
nichtionogene Tenside, z.B. polyoxyethyliertes Rizinusöl, polyoxyethyliertes Sorbitanmonooleat, Sorbitanmonostearat, Glycerinmonostearat, Polyoxyethylstearat, Alkylphenolpolyglykolether;
ampholytische Tenside wie Di-Na-N-lauryl-$\beta$-iminodipropionat oder Lecithin;
anionaktive Tenside, wie Na-Laurylsulfat, Fettalkoholethersulfate, Mono/Dialkylpolyglykoletherorthophosphorsäureester-monoethanolaminsalz;
kationaktive Tenside wie Cetyltrimethylammoniumchiorid.

Als weitere Hilfsstoffe seien genannt:
Viskositätserhöhende und die Emulsion stabilisierende Stoffe wie Carboxymethylcellulose, Methylcellulose und andere Cellulose- und Stärke-Derivate, Polyacrylate, Alginate, Gelatine, Gummi-arabicum, Polyvinylpyrrolidon, Polyvinylalkohol, Copolymere aus Methylvinylether und Maleinsäureanhydrid, Polyethylenglykole, Wachse, kolloidale Kieselsäure oder Gemische der aufgeführten Stoffe.

Suspensionen können oral, dermal oder als Injektion angewendet werden. Sie werden hergestellt, indem man den Wirkstoff in einer Trägerflüssigkeit gegebenenfalls unter Zusatz weiterer Hilfsstoffe wie Netzmittel, Farbstoffe, resorptionsfördernde Stoffe, Konservierungsstoffe, Antioxidantien, Lichtschutzmittel suspendiert.

Als Trägerflüssigkeiten seien alle homogenen Lösungsmittel und Lösungsmittelgemische genannt.

Als Netzmittel (Dispergiermittel) seien die weiter oben angegebenen Tenside genannt.

Als weitere Hilfsstoffe seien die weiter oben angegebenen genannt.

Halbfeste Zubereitungen können oral oder dermal verabreicht werden. Sie unterscheiden sich von den oben beschriebenen Suspensionen und Emulsionen nur durch ihre höhere Viskosität.

Zur Herstellung fester Zubereitungen wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Alle solche dienen anorganische und organische Stoffe. Anorganische Stoffe sind z.B. Kochsalz, Carbonate wie Calciumcarbonat, Hydrogencarbonate, Aluminiumoxide, Kieselsäuren, Tonerden, gefälltes oder kolloidales Siliciumdioxid, Phosphate.

Organische Stoffe sind z.B. Zucker, Zellulose, Nahrungs- und Futtermittel wie Milchpulver, Tiermehle, Getreidemehle und -schrote, Stärken.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite, zerfallsfördernde Substanzen wie Stärke oder quervernetztes Polyvinylpyrrolidon, Bindemittel wie z.B. Stärke, Gelatine oder lineares Polyvinylpyrrolidon sowie Trockenbindemittel wie mikrokristalline Cellulose.

Die Wirkstoffe können in den Zubereitungen auch in Mischung mit Synergisten oder mit anderen Wirkstoffen vorliegen.

Anwendungsfertige Zubereitungen enthalten den Wirkstoff in Konzentrationen von 10 ppm bis 20 Gewichtsprozent, bevorzugt von 0,1 bis 10 Gewichtsprozent.

Zubereitungen die vor Anwendung verdünnt werden, enthalten den Wirkstoff in Konzentrationen von 0,5 bis 90 Gewichtsprozent, bevorzugt von 1 bis 50 Gewichtsprozent.

Im allgemeinen hat es sich als vorteilhaft erwiesen, Mengen von etwa 0,5 bis etwa 50 mg, bevorzugt 1 bis 20 mg, Wirkstoff je kg Körpergewicht pro Tag zur Erzielung wirksamer Ergebnisse zu verabreichen.

Die Wirkstoffe können auch zusammen mit dem Futter oder Trinkwasser der Tiere verabreicht werden.

Futter- und Nahrungsmittel enthalten 0,01 bis 250 ppm, vorzugsweise 0,5 bis 100 ppm des Wirkstoffs in Kombination mit einem geeigneten eßbaren Material.

Ein solches Futter- und Nahrungsmittel kann sowohl für Heilzwecke als auch für prophylaktische Zwecke versendet werden.

Die Herstellung eines solchen Futter- oder Nahrungsmittels erfolgt durch Mischen eines Konzentrats oder einer Vormischung, die 0,5 bis 30 %, vorzugsweise 1 bis 20 Gew.-% eines Wirkstoffs in Mischung mit einem eßbaren organischen oder anorganischen Träger enthält mit üblichen Futtermitteln. Eßbare Träger sind z.B. Maismehl oder Mais- und Sojabohnenmehl oder Mineralsalze, die vorzugsweise eine geringe Menge eines eßbaren Staubverhütungsöls, z.B. Maisöl oder Sojaöl, enthalten. Die hierbei erhaltene Vormischung kann darin dem vollständigen Futtermittel vor seiner Verfütterung an die Tiere zugesetzt werden.

Beispielhaft sei der Einsatz bei der Coccidiose genannt:

Für die Heilung und Prophylaxe etwa der Coccidiose bei Geflügel, insbesondere bei Hühnern, Enten, Gänsen und Truthähnen, werden 0,1 bis 100 ppm, vorzugsweise 0,5 bis 100 ppm eines Wirkstoffs mit einem geeigneten eßbaren Material, z.B. einem nahrhaften Futtermittel, gemischt. Falls gewünscht, können diese Mengen erhöht werden, besonders wenn der Wirkstoff vom Empfänger gut vertragen wird. Entsprechend kann die Verabreichung über das Trinkwasser erfolgen.

Für die Behandlung von Einzeltieren, z.B. im Falle der Behandlung der Coccidiose bei Säugetieren oder der Toxoplasmose, werden vorzugsweise Wirkstoffmengen von 0,5 bis 100 mg/kg Körpergewicht täglich verabreicht, um die gewünschten Ergebnisse zu erzielen. Trotzdem kann es zeitweilig notwendig sein, von den genannten Mengen abzuweichen, insbesondere in Abhängigkeit vom Körpergewicht des Versuchstieres oder der Art der Verabreichungsmethode, aber auch wegen der Tiergattung und seiner individuellen Reaktion auf den Wirkstoff oder der Art der Formulierung und der Zeit oder dem Abstand, zu dem er verabreicht wird. So kann es in gewissen Fällen genügen, mit weniger als der vorstehend genannten Mindestmenge auszukommen, während in anderen Fällen die genannte obere Grenze überschritten werden muß. Bei der Verabreichung größerer Mengen kann es zweckmäßig sein, diese im Verlauf des Tages in mehrere Einzeldarreichungen zu unterteilen.

Darüber hinaus sind die erfindungsgemäßen Verbindungen wirksam gegenüber verschiedenen zu den Helminthen (Würmern) zählenden Fischparasiten.

Zu den Parasiten bei Fischen gehören aus dem Unterreich der Protozoen Spezies des Stammes der Ciliata, z.B. Ichthyophthirius multifiliis, Chilodonella cyprini, Trichodina spp., Glossatella spp., Epistylis spp. des Stammes der Myxosporidia, z.B. Myxosoma cerebralis, Myxidium spp., Myxobolus spp., Heneguya spp., Hoferellus spp., der Klasse der Mikrosporidia z.B. Glugea spp., Thelohania spp., Pleistophora spp., aus dem Stamm der Plathelminthen: Trematoden; Monogenea z.B. Dactylogyrus spp., Gyrodactylus spp., Pseudodactylogyrus spp., Diplozoon spp., Cestoden, z.B. aus den Gruppen der Caryphyllidea (z.B. Caryophyllaeus laticeps), Pseudophyllidea (z.B. Diphyllobothrium spp.), Tetraphyllidea (z.B. Phyllobothrium spp.) und Protocephalida (z.B. Arten der Gattung Proteocephalus) und aus dem Stamm der Arthropoda verschiedene parasitische Crustaceen, insbesondere aus den Unterklassen der Branchiura (Fischläuse) und Copepoda (Ruderfußkrebse) sowie den Ordnungen der Isopoda (Asseln) und Amphipoda (Flohkrebse).

Die Behandlung der Fische erfolgt entweder oral, z.B. über das Futter oder durch Kurzzeitbehandlung, "medizinisches Bad", in das die Fische eingesetzt und in dem sie eine Zeitlang (Minuten bis mehrere Stunden) z.B. beim Umsetzen von einem Zuchtbecken zum anderen gehalten werden.

Es kann aber auch eine vorübergehende oder dauernde Behandlung des Lebensraums der Fische (z.B. ganzer Teichanlagen, Aquarien, Tanks oder Becken), in denen die Fische gehalten werden, erfolgen.

Der Wirkstoff wird in Zubereitungen verabreicht, die den Anwendungen angepaßt sind.

Die Konzentration des Wirkstoffs liegt in den Zubereitungen bei 1 ppm bis 10 Gew.-%.

Bevorzugte Zubereitungen zur Kurzzeitbehandlung in der Anwendung als "medizinisches Bad" z.B. bei der Behandlung beim Umsetzen der Fische oder zur Behandlung des Lebensraums (Teichbehandlung) der Fische sind Lösungen des Wirkstoffs in einem oder mehreren polaren Lösungsmitteln, die bei Verdünnen mit Wasser alkalisch reagieren.

Zur Herstellung dieser Lösungen wird der Wirkstoff in einem polaren, wasserlöslichen Lösungsmittel gelöst, welches entweder alkalisch reagiert oder dem eine alkalische wasserlösliche Substanz zugefügt wird. Letztere wird vorteilhaft ebenfalls im Lösungsmittel gelöst, kann aber auch in dem Lösungsmittel

suspendiert sein und sich erst im Wasser lösen. Dabei soll das Wasser nach Zusatz der Wirkstofflösung einen pH-Wert von 7 bis 10, vorzugsweise aber einen pH-Wert von 8 bis 10 haben.

Die Konzentration des Wirkstoffes kann im Bereich von 0,5 bis 50 % liegen, vorzugsweise aber in einem Bereich von 1 bis 25 %.

Als Lösungsmittel kommen alle wasserlöslichen Lösungsmittel in Betracht, in denen der Wirkstoff in genügender Konzentration löslich ist und die physiologisch unbedenklich sind.

Dies sind Ethylalkohol, Isopropylalkohol, Benzylalkohol, Glycerin, Propylenglykol, Polyethylenglykole, Poly(oxoethylen)-poly(oxypropylen)-Polymere, basische Alkohole wie Mono-, Di- und Triethanolamin, Ketone wie Aceton oder Methylethylketon, Ester wie Milchsäureethylester, ferner N-Methylpyrrolidon, Dimethylacetamid, Dimethylformamid, ferner Dispergier- und Emulgiermittel wie polyoxyethyliertes Rizinusöl, Polyethylenglykol-Sorbitan-Monooleat, Polyethylenglykolstearat, oder Polyethylenglykolether, Polyethylenglykol-Alkylamine.

Als Basen zur Einstellung des alkalischen pH-Wertes seien genannt organische Basen wie basische Aminosäuren wie L- bzw. D, L-Arginin, L- bzw. D, L-Lysin, Methylglucoseamin, 2-Amino-2-hydroxymethylpropandiol-(1,3) ferner wie N,N,N',N'-tetrakis-(2-hydroxypropyl)-ethylendiamin oder Polyether-Tetrol auf der Basis Ethylendiamin (M.G. 480-420), anorganische Basen, wie Ammoniak oder Natriumcarbonatgegebenenfalls unter Zugabe von Wasser.

Die Zubereitungen können auch 0,1 bis 20 Gew.-%, vorzugsweise 0,1 bis 10 Gew.-% anderer Formulierhilfsstoffe wie Antioxydantien, Tenside, Suspensionsstabilisatoren und Verdickungsmittel wie z.B. Methylcellulose, Alginate, Polysaccharide, Galaktomannane und kolloidale Kieselsäure enthalten. Der Zusatz von Farbe, Aroma und Aufbaustoffen zur Tierernährung ist ebenfalls möglich. Auch Säuren, die mit der vorgelegten Base zusammen ein Puffersystem bilden oder den pH der Lösung reduzieren, sind hier zu nennen.

Die Konzentration des Wirkstoffs bei der Anwendung hängt ab von Art und Dauer der Behandlung, sowie Alter und Zustand der behandelten Fische. Sie beträgt z.B. bei Kurzzeitbehandlung 2 bis 50 mg Wirkstoff pro Liter Wasser, bevorzugt 5 bis 10 mg pro Liter, bei einer Behandlungsdauer von 3 bis 4 Stunden. Bei der Behandlung von jungen Karpfen wird z.B. mit einer Konzentration von 5 bis 10 mg/l und einer Behandlungsdauer von ca 1 bis 4 Stunden gearbeitet.

Aale werden mit Konzentrationen von ca. 5 mg/l ca. 4 Stunden behandelt.

Bei längerer Behandlungsdauer oder bei Dauerbehandlung kann die Konzentration entsprechend niedriger gewählt werden.

Bei Teichbehandlungen können 0,1 bis5 mg Wirkstoff pro Liter Wasser verwendet werden.

Zubereitungen zur Anwendung als Futterzusatz sind z.B. wie folgt zusammengesetzt:

a)

| | | |
|---|---|---|
| Wirkstoff der Formel I | 1 - 10 | Gewichtsteile |
| Sojabohnen-Protein | 49 - 90 | Gewichtsteile |

b)

| | | |
|---|---|---|
| Wirkstoff der Formel I | 0,5 - 10 | Gewichtsteile |
| Benzylalkohol | 0,08 - 1,4 | Gewichtsteile |
| Hydroxypropylmethylcellulose | 0 - 3,5 | Gewichtsteile |
| Wasser | | Rest ad 100 |

Zubereitungen zur Anwendung bei "medizinischen Bädern" und zur Teichbehandlung sind z.B. wie folgt zusammengesetzt und hergestellt.

c) 2,5 g Wirkstoff der Formel (I) werden in 100 ml Triethanolamin unter Erwärmen gelöst.

d) 2,5 g Wirkstoff der Formel (I)

12,5g Milchsäure werden in 100 ml Triethanolamin unter Erwärmen und Rühren gelöst

e) 10,0g Wirkstoff der Formel (I) wird in 100 ml Monoethanolamin gelöst.

f)

| | |
|---|---|
| Wirkstoff der Formel I | 5,0 g |
| Propylenglykol | 50,0 g |
| Natriumcarbonat | 5,0 g |
| Wasser | ad 100 ml |

g)

| | |
|---|---|
| Wirkstoff der Formel I | 5,0 g |
| Monoethanolamin | 10 g |
| N-Methylpyrrolidon | ad 100 ml |

h)

| | |
|---|---|
| Wirkstoff der Formel I | 2,5g |
| Natriumcarbonat | 5,0 g |
| Polyethylenglykol 200 | ad 100 ml |

Der Wirkstoff wird unter Erwärmen im Polyethylenglykol gelöst und Natriumcarbonat darin suspendiert.

**Beispiel A**

Coccidiose bei Hühnern

9 bis 11 Tage alte Küken wurden mit 40 000 sporulierten Oozysten von stark virulenten Stämmen von Eimeria acervuline, E. maxima und E. tenella, den Krankheitserregern der intestinalen Coccidiose infiziert.

3 Tage vor der Infektion und 8 Tage nach der Infektion (Ende des Versuchs) wurde Wirkstoff in der angegebenen Konzentration im Futter der Tiere eingemischt verabreicht.

Die Zahl der Oozysten im Kot wurde mit Hilfe der McMaster-Kammer bestimmt [siehe Engelbrecht und Mitarbeiter "Parasitologische Arbeitsmethoden in Medizin und Veterinärmedizin", S. 172, Akademie-Verlag, Berlin (1965)].

In der folgenden Tabelle werden Wirkstoffdosen und Oozystenausscheidung in % für die einzelnen Erreger angegeben. Dabei bedeutet 100 % keine Wirkung und 0 % volle Wirkung, d.h. keine Oozystenausscheidung.

Tabelle 1

| Coccidiose bei Hühnern | | | | |
|---|---|---|---|---|
| Beispiel Nr. | Dosis ppm | E.acervulina Oocystenausscheidung in % im Vergleich zur unbehandelten infizierten Kontrolle | E. maxima Oocytenausscheidung in % im Vergleich zur nicht infizierten unbehandelten Kontrolle | E. tenella |
| unbehandelte infizierte Kontrolle | 0 | 100 | 100 | 100 |
| 37 | 250<br>100 | 0,5<br>28 | 0<br>26 | 0<br>32 |
| 45 | 250<br>100 | 0,1<br>11 | 0<br>8 | 0<br>5 |
| 57 | 250<br>100 | 0,01<br>38 | 0<br>5,3 | 0<br>93 |

**Herstellungsbeispiele:**

Beipiel 1:

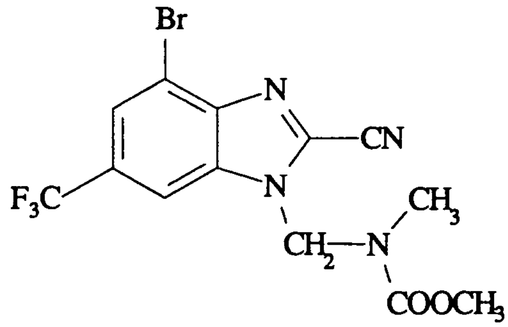

Eine Mischung aus 9,0 g (0,03 Mol) 4-Brom-2-cyan-6-trifluormethyl-1H-benzimidazol (vergl. z.B. EP 239 508), 8,3 g (0,06 Mol) Kaliumcarbonat, 4,2 g (0,03Mol) N-Chlormethyl-N-methyl-carbamidsäuremethylester und 105 ml Essigester wird 4 Stunden auf Rückflußtemperatur erhitzt. Zur Aufarbeitung wird die abgekühlte Reaktionsmischung filtriert, das Filtrat mit Wasser gewaschen, über Natriumsulfat getrocknet und im Vakuum eingeengt. Der Rückstand läßt sich durch Umkristallisieren aus Ether/Petrolether (1:10) und anschließende Säulenchromatographie an Kieselgel (Laufmittel: Dichlormethan) reinigen.

Man erhält 6,0 g (51% der Theorie) an N-(4-Brom-2-cyano-6-trifluormethyl-1H-benzimidazol-1-yl-methyl)-N-methyl-carbamidsäuremethylester vom Schmelzpunkt 138-141 °C.

In entsprechender Weise und gemäß den allgemeinen Angaben zur Herstellung erhält man die folgenden substituierten Benzimidazole der allgemeinen Formel (I):

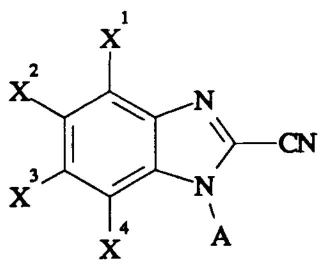

(I)

| Bsp.-Nr. | $X^1$ | $X^2$ | $X^3$ | $X^4$ | A | physikalische Eigenschaften |
|---|---|---|---|---|---|---|
| 2 | H | $NO_2$<br>(H) | H<br>($NO_2$) | H | 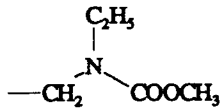 | Fp. 120-121°C<br>(69:31) |
| 3 | H | $NO_2$<br>(H) | H<br>($NO_2$) | H | 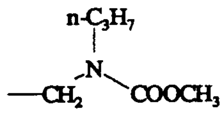 | Fp. 99-102°C<br>(65:35) |
| 4 | H | $NO_2$<br>(H) | H<br>($NO_2$) | H | 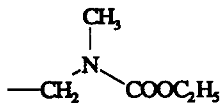 | Fp. 92-96°C<br>(59:41) |
| 5 | H | $NO_2$<br>(H) | H<br>($NO_2$) | H | 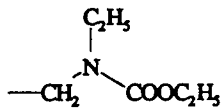 | Fp. 65-70°C<br>(59:41) |
| 6 | H | $NO_2$<br>(H) | H<br>($NO_2$) | H | 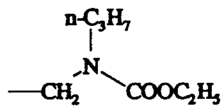 | $^1$H-NMR*):<br>6,0; 6,05<br>(72:28) |
| 7 | H | $NO_2$<br>(H) | H<br>($NO_2$) | H | 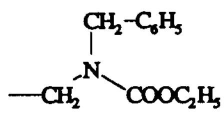 | Fp. 78-82°C |
| 8 | Br | H | $CF_3$ | H | 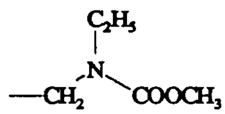 | Fp. 107-110°C |

| Bsp.-Nr. | $X^1$ | $X^2$ | $X^3$ | $X^4$ | A | physikalische Eigenschaften |
|---|---|---|---|---|---|---|
| 9 | Br | H | $CF_3$ | H | 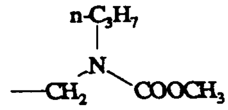 | $^1H$-NMR*): 6,03 |
| 10 | H | $CF_3$ | H | Br | 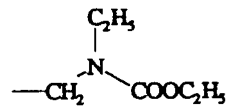 | Fp. 163-168°C |
| 11 | Br | H | $CF_3$ | H | 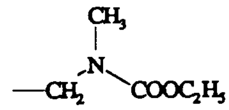 | Fp. 110-113°C |
| 12 | Br | H | $CF_3$ | H | 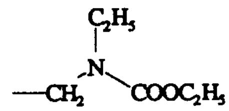 | Fp. 84-87°C |
| 13 | Br | H | $CF_3$ | H | 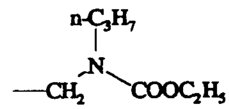 | $^1H$-NMR*): 6,02 |
| 14 | H | $NO_2$ (H) | H ($NO_2$) | H | 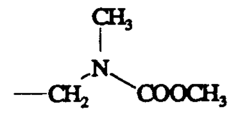 | Fp. 127-131°C (65:31) |
| 15 | Br | H | $CF_3$ | H | $-CH_2-COOCH_3$ | Fp. 160-163°C |
| 16 | Br | H | $CF_3$ | H | $-CH_2-COOC_2H_5$ | Fp. 113-117°C |
| 17 | Br | H | $CF_3$ | H | 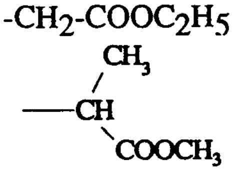 | Fp. 105-108°C |
| 18 | Br | H | $CF_3$ | H | $-CH_2-NH-COOCH_3$ | Fp. 205-208°C |
| 19 | Br | H | $CF_3$ | H | $-CH_2-CN$ | Fp. 157-160°C |
| 20 | H | $NO_2$ (H) | H ($NO_2$) | H | 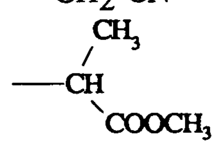 | Fp. 87-91°C (57:43) |
| 21 | H | $NO_2$ (H) | H ($NO_2$) | H | $-CH_2-COOC_2H_5$ | Fp. 96-100°C (1:1) |
| 22 | H | $NO_2$ (H) | H ($NO_2$) | H | $-CH_2-COOCH_3$ | Fp. 141-143°C (1:1) |

| Bsp.-Nr. | $X^1$ | $X^2$ | $X^3$ | $X^4$ | A | physikalische Eigenschaften |
|---|---|---|---|---|---|---|
| 23 | Br | H | $CF_3$ | H | 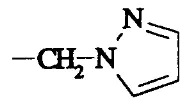 | Fp. 154-157°C |
| | (H) | ($CF_3$) | (H) | (Br) | | (63:37) |
| 24 | Br | H | $CF_3$ | H | 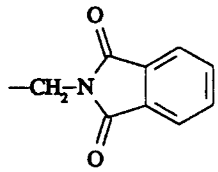 | Fp. 212-217°C |
| 25 | Br | H | $CF_3$ | H | $-CH_2-CH=CH_2$ | Fp. 95-99°C |
| | (H) | ($CF_3$) | (H) | (Br) | | (78:22) |
| 26 | H | $CF_3$ | H | Br | $-CH_2-CN$ | Fp. 170-172°C |
| 27 | Br | H | $CF_3$ | H | 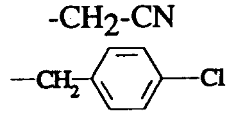 | Fp. 147-150°C |
| | (H) | ($CF_3$) | (H) | (Br) | | (86:14) |
| 28 | Br | H | $CF_3$ | H | $-CH_2-C_6H_5$ | Fp. 140-143°C |
| | (H) | ($CF_3$) | (H) | (Br) | | (95:5) |
| 29 | Br | H | $CF_3$ | H | $-CH_2-COOH$ | Fp. >195°C (Z) |
| 30 | Br | H | $CF_3$ | H | 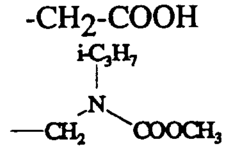 | Fp. 83-86°C |
| 31 | Br | H | $CF_3$ | H | 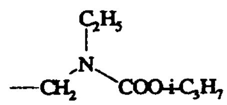 | Fp. 90-93°C |
| 32 | Br | H | $CF_3$ | H | 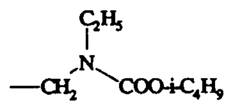 | Fp. 97-101°C |
| 33 | Br | H | $CF_3$ | H | 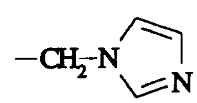 | Fp. 120-123°C |
| | (H) | ($CF_3$) | (H) | (Br) | | (84:16) |
| 34 | Br | H | $CF_3$ | H | 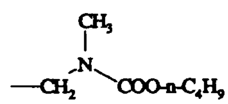 | Fp. 101-105°C |
| 35 | Br | H | $CF_3$ | H | 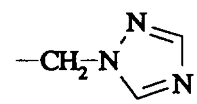 | Fp. 160-163°C |
| | (H) | ($CF_3$) | (H) | (Br) | | (75:25) |
| 36 | Br | H | $CF_3$ | H | 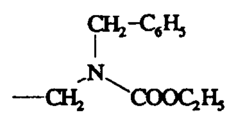 | $^1$H-NMR*): 6,11 |

| Bsp.-Nr. | $X^1$ | $X^2$ | $X^3$ | $X^4$ | A | physikalische Eigenschaften |
|---|---|---|---|---|---|---|
| 37 | Br | H | $CF_3$ | H | 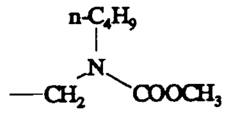 | $^1$H-NMR*): 5,94 |
| 38 | Br | H | $CF_3$ | H | 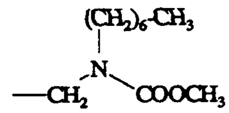 | $^1$H-NMR*): 6,06 |
| 39 | Br | H | $CF_3$ | H | 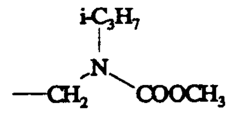 | Fp. 86-90°C |
| 40 | Br | H | $CF_3$ | H | 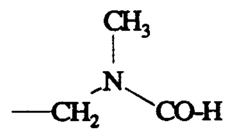 | Fp. 137-140°C |
| 41 | Br (H) | H ($CF_3$) | $CF_3$ (H) | H (Br) | -O-n-$C_3H_7$ | Fp. 105-109°C (86:14) |
| 42 | Br (H) | H ($CF_3$) | $CF_3$ (H) | H (Br) | -O-i-$C_3H_7$ | $^1$H-NMR*): 5,98; 6,06 (53:47) |
| 43 | Br (H) | H ($CF_3$) | $CF_3$ (H) | H (Br) | -O--$CH_2$-C≡CH | Fp. 73-76°C (53:47) |
| 44 | Br (H) | H ($CF_3$) | $CF_3$ (H) | H (Br) | 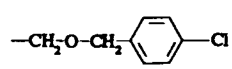 | Fp. 75-78°C (36:64) |
| 45 | Br (H) | H ($CF_3$) | $CF_3$ (H) | H (Br) | -$CH_2$-O-CO-t-$C_4H_9$ | $^1$H-NMR*): 6,49 (66:34) |
| 46 | Br (H) | H ($CF_3$) | $CF_3$ (H) | H (Br) | -$CH_2$-O-$(CH_2)_3$-$C_6H_5$ | $^1$H-NMR*): 5,96; 6,05 |
| 47 | Br | H | $CF_3$ | H | 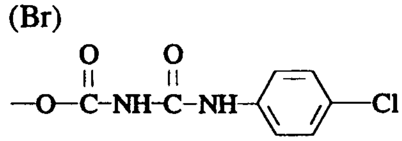 | Fp. 175-180°C |

| Bsp.-Nr. | $X^1$ | $X^2$ | $X^3$ | $X^4$ | A | physikalische Eigenschaften |
|---|---|---|---|---|---|---|
| 48 | Br<br>(H) | H<br>($CF_3$) | $CF_3$<br>(H) | H<br>(Br) | 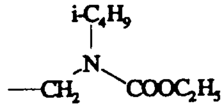 | $^1$H-NMR*):<br>6,03<br>(76:24) |
| 49 | Br | H | $CF_3$ | H | 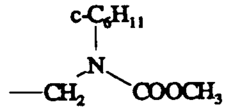 | Fp. 90-93°C |
| 50 | Br | H | $CF_3$ | H | 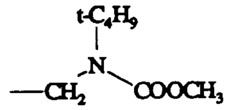 | Fp. 105-108°C |
| 51 | Br<br>(H) | H<br>($CF_3$) | $CF_3$<br>(H) | H<br>(Br) | 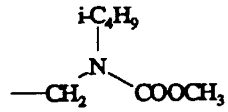 | Fp. 86-90°C<br>(81:19) |
| 52 | Br<br>(H) | H<br>($CF_3$) | $CF_3$<br>(H) | H<br>(Br) | 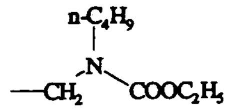 | $^1$H-NMR*):<br>5,95; 6,35<br>(80:20) |
| 53 | Br | H | $CF_3$ | H | 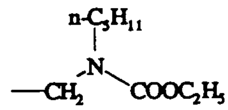 | $^1$H-NMR*):<br>6,05 |
| 54 | Br<br>(H) | H<br>($CF_3$) | $CF_3$<br>(H) | H<br>(Br) | 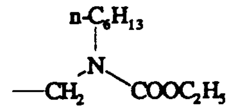 | $^1$H-NMR*):<br>5,95<br>(76:24) |
| 55 | Br<br>(H) | H<br>($CF_3$) | $CF_3$<br>(H) | H<br>(Br) | 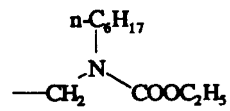 | $^1$H-NMR*):<br>5,95; 6,35<br>(72:28) |
| 56 | Br | H | $CF_3$ | H | 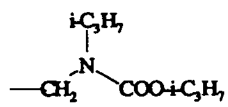 | Fp. 115-120°C |
| 57 | Br | H | $CF_3$ | H | 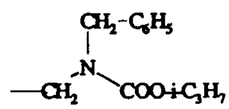 | Fp. 112-116°C |
| 58 | Br | H | $CF_3$ | H | 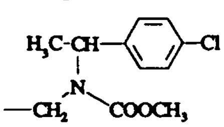 | Fp. 135-140°C |
| 59 | H | H | $CF_3$ | H | $-O-C_2H_5$ | Fp. 173-176°C |

| Bsp.-Nr. | $X^1$ | $X^2$ | $X^3$ | $X^4$ | A | physikalische Eigenschaften |
|---|---|---|---|---|---|---|
| 60 | H | H | $F_2CH-CF_2-O-$ | H | $-O-C_2H_5$ | Fp. 35-40°C |
| 61 | H | $CF_3-O-$ | $CF_3-O-$ | H | $-O-C_2H_5$ | $^1$H-NMR*): 5,91 |
| 62 | H | $C_6H_5$ (H) | H ($C_6H_5$) | H | $-O-C_2H_5$ | $^1$H-NMR*): 5,87; 5,92 |
| 63 | H | $-O-(CH_2)_2-O-$ | | H | $-O-C_2H_5$ | Fp. 150-151°C |
| 64 | H | $-O-(CH_2)_2-O-$ | | H | 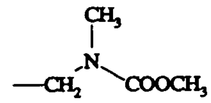 | Fp. 162-164°C |
| 65 | H | $-O-CF_2-CFCl-O-$ ($-O-CFCl-CF_2-O-$) | | H | 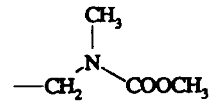 | Fp. 127-130°C |
| 66 | H | $-O-CF_2-CFCl-O-$ ($-O-CFCl-CF_2-O-$) | | H | $-O-C_2H_5$ | Fp. 96-100°C (1:1) |
| 67 | H | $-O-CF_2-CFCl-O-$ ($-O-CFCl-CF_2-O-$) | | H | 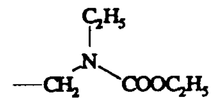 | $^1$H-NMR*): 5,91 |
| 68 | H | $-O-CF_2-CFCl-O-$ ($-O-CFCl-CF_2-O-$) | | H | 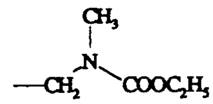 | Fp. 130-133°C |
| 69 | H | $-O-CF_2-CFCl-O-$ ($-O-CFCl-CF_2-O-$) | | H | 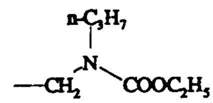 | Fp. 62-66°C |
| 70 | H | $-O-CF_2-CF_2-O-$ | | H | $-O-C_2H_5$ | Fp. 90-93°C |
| 71 | H | $-O-CF_2-CF_2-O-$ | | H | 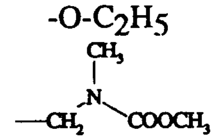 | Fp. 140-144°C |
| 72 | H | $-O-CF_2-CF_2-O-$ | | H | $-CH_2-O-(CH_2)_2-OCH_3$ | Fp. 60-64°C |
| 73 | H | $-O-CF_2-CF_2-O-$ | | H | 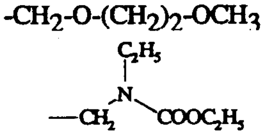 | Fp. 53-57°C |
| 74 | H | $-O-CF_2-CF_2-O-$ | | H | 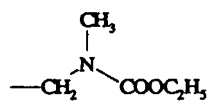 | Fp. 123-125°C |

| Bsp.-Nr. | $X^1$ | $X^2$ | $X^3$ | $X^4$ | A | physikalische Eigenschaften |
|---|---|---|---|---|---|---|
| 75 | H | $-O-CF_2-CF_2-O-$ | | H | 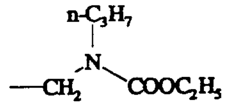 | Fp. 70-73°C |
| 76 | H | $-O-CF_2-CHF-O-$ | | H | $-O-C_2H_5$ | Fp. 75-80°C |
| 77 | H | $-O-CF_2-O-$ | | H | $-O-C_2H_5$ | Fp. 110-113°C |
| 78 | H | $-O-CF_2-O-$ | | H | 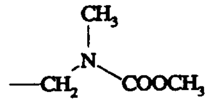 | Fp. 147-150°C |

*) Die $^1H$-NMR-Spektren wurden in Deuterochloroform ($CDCl_3$) oder Hexadeutero-Dimethylsulfoxid (DMSO-$d_6$) mit Tetramethylsilan (TMS) als innerem Standard aufgenommen. Angegeben ist die chemische Verschiebung der $N-CH_2-N$-Gruppe als δ-Wert in ppm.

**Herstellung der Ausgangsverbindungen:**

Beispiel II-1:

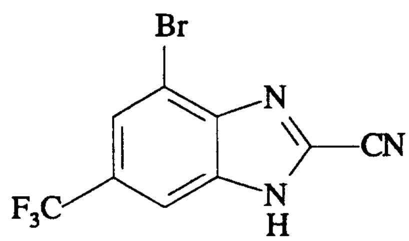

Zu 900 ml gesättigter wässriger Ammoniaklösung gibt man bei 15 ° C bis 25 ° C tropfenweise unter Rühren eine Lösung von 120 g (0,3 Mol) 4-Brom-2-trichlormethyl-6-trifluormethyl-1H-benzimidazol (vergl. z.B. EP 239 508) in 300 ml Ethanol und rührt nach beendeter Zugabe eine Stunde bei Raumtemperatur. Zur Aufarbeitung wird die Reaktionsmischung mit 4.000 ml 20-prozentiger Salzsäure auf pH 1 gebracht und dreimal mit jeweils 300 ml Dichlormethan extrahiert. Die vereinigten organischen Phasen werden mit Natriumsulfat getrocknet, im Vakuum eingeengt und das verbleibende Öl durch Chromatographie an Kieselgel (Laufmittel: Cyclohexan/Essigester 2:1) und Kristallisation aus Petrolether gereinigt.

Man erhält 49,1 g (55% der Theorie) an 4-Brom-2-cyano-6-trifluormethyl-1H-benzimidazol vom Schmelzpunkt 130 - 134 ° C.

In entsprechender Weise erhält man die folgenden substituierten 1H-Benzimidazole der allgemeinen Formel (II):

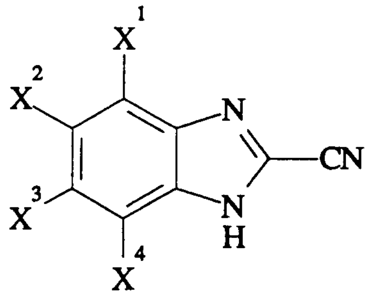

(II)

| Bsp.-Nr. | $X^1$ | $X^2$ | $X^3$ | $X^4$ | physikalische Eigenschaften |
|---|---|---|---|---|---|
| II-2 | H | $NO_2$ (H) | H ($NO_2$) | H | Fp. >240°C |
| II-3 | H | $-O-CF_2-CFCl-O-$ ($-O-CFCl-CF_2-O-$) | | H | Fp. >230°C |
| II-4 | H | $-O-CF_2-CF_2-O-$ | | H | Fp. >230°C |
| II-5 | H | H ($CF_3$) | $CF_3$ (H) | H | Fp. 173-176°C |
| II-6 | H | H ($C_2H_5-O-$) | $C_2H_5-O-$ (H) | H | Fp. 86-90°C |
| II-7 | H | H ($F_2CH-CF_2-O-$) | $F_2CH-CF_2-O-$ (H) | H | Fp. 130-132°C |
| II-8 | H | $CF_3-O-$ | $CF_3-O-$ | H | Fp. 194-198°C |
| II-9 | H | H ($C_6H_5$) | $C_6H_5$ (H) | H | Fp. >230°C |
| II-10 | H | $-O-(CH_2)_3-O-$ | | H | Fp. >230°C |

| Bsp.-Nr. | $X^1$ | $X^2$ | $X^3$ | $X^4$ | physikalische Eigenschaften |
|---|---|---|---|---|---|
| II-11 | H | $-O-CF_2-CHF-O-$ ($-O-CHF-CF_2-O-$) | | H | Fp. 50-60°C |
| II-12 | H | $-O-CF_2-O-$ | | H | Fp. >220°C |

**Patentansprüche**

**1.** Verwendung von CN-substituierten Benzimidazolen der allgemeinen Formel (I)

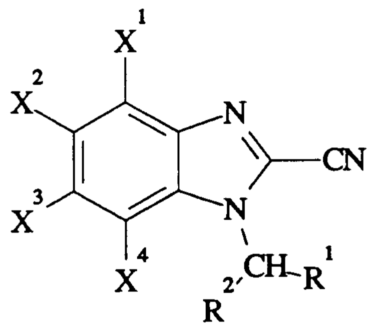

(I)

in welcher

| | |
|---|---|
| $X^1$, $X^2$, $X^3$ und $X^4$ | unabhängig voneinander jeweils für Wasserstoff, Halogen, Cyano, Nitro, für jeweils gegebenenfalls substituiertes Alkyl, Alkoxy, Alkylthio, Alkylsulfinyl, Alkylsulfonyl oder Cycloalkyl, für gegebenenfalls substituiertes, ankondensiertes Dioxyalkylen, für Hydroxycarbonyl, Alkylcarbonyl, Alkoxycarbonyl, Cycloalkyloxycarbonyl, für jeweils gegebenenfalls substituiertes Amino oder Aminocarbonyl oder für jeweils gegebenenfalls substituiertes Aryl, Aryloxy, Arylthio, Arylsulfinyl, Arylsulfonyl, Arylsulfonyloxy, Arylcarbonyl, Aryloxycarbonyl, Arylazo oder Arylthiomethylsulfonyl stehen, wobei jedoch mindestens einer der Substituenten$X^1$, $X^2$, $X^3$ und $X^4$ verschieden von Wasserstoff und Halogen ist und wobei |
| $R^1$ | für Wasserstoff, Alkyl oder für gegebenenfalls substituiertes Aryl steht, |
| $R^2$ | für Hydroxy, Cyano oder für jeweils gegebenenfalls substituiertes Alkyl, Alkenyl, Alkinyl, Alkoxy, Alkenyloxy, Alkinyloxy, Alkylthio, Amino, Alkylcarbonyl, Alkoxycarbonyl, Alkylcarbonyloxy, Dialkoxyphosphoryl, (Hetero)Aryl, (Hetero)-Arylcarbonyl, (Hetero)Aryloxycarbonyl, (Hetero)Arylcarbonyloxy oder (Hetero)-Arylaminocarbonylaminocarbonyloxy steht, |

als Mittel zur Bekämpfung parasitärer Protozoen und insbesondere Coccidien.

2. Mittel gegen parasitäre Protozoen, gekennzeichnet durch einen Gehalt an mindestens einem substituierten Benzimidazol der Formel (I) gemäß Anspruch 1.

3. Verfahren zur Bekämpfung von parasitären Protozoen, dadurch gekennzeichnet, daß man substituierte Benzimidazole der Formel (I) gemäß Anspruch 1 auf diese und/oder ihren Lebensraum einwirken läßt.

4. Verfahren zur Herstellung von Mitteln gegen parasitäre Protozoen, dadurch gekennzeichnet, daß man substituierte Benzimidazole der Formel (I) gemäß Anspruch 1 mit Streckmitteln und/oder oberflächenaktiven Mitteln vermischt.

5. Verwendung von substituierten Benzimidazolen der Formel (I) gemäß Anspruch 1 zur Herstellung von Mitteln gegen parasitäre Protozoen.

Europäisches Patentamt

# EUROPÄISCHER RECHERCHENBERICHT

Nummer der Anmeldung
EP 93 11 9425

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
|---|---|---|---|
| D,X | DE-A-20 14 923 (FISONS PEST CONTROL LTD.)<br>* Seite 1 - Seite 5 *<br>* Anspruch 5 * | 2,4 | A01N43/52<br>A61K31/415 |
| D,A | DE-A-20 47 369 (MERCK& CO. INC.)<br>* Seite 1 - Seite 4, Absatz 1 *<br>* Seite 19 - Seite 20, Absatz 1 *<br>* Anspruch 1 * | 1-5 | |
| A | GB-A-1 022 659 (S.B.PERNICK & COMPANY)<br>* Seite 1, Zeile 6 - Seite 2, Zeile 16 * | 1-5 | |
| A | DE-A-27 36 448 (FISONS LTD.)<br>* Seite 3 - Seite 5, Absatz 2 * | 1-5 | |
| A | FR-A-1 476 531 (CHIMETRON S.A.R.L.)<br>* Seite 1, linke Spalte, Absatz 2 - rechte Spalte, Absatz 2 *<br>* Seite 2, linke Spalte, Absatz 2 -Absatz 5 *<br>* Ansprüche 1,3,4 * | 1-5 | **RECHERCHIERTE SACHGEBIETE (Int.Cl.5)** |
| A | FR-A-1 439 128 (CHIMETRON S.A.R.L.)<br>* Seite 1, linke Spalte, Absatz 2 - rechte Spalte, vorletzter Absatz *<br>* Ansprüche 1-3 * | 1-5 | A01N<br>A61K<br>C07D |

Der vorliegende Recherchenbericht wurde für alle Patentansprüche erstellt

| Recherchenort | Abschlußdatum der Recherche | Prüfer |
|---|---|---|
| DEN HAAG | 17. März 1994 | Muellners, W |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)